# DEMANDE DE BREVET EUROPEEN

(11) **EP 1 225 170 A2**
(43) Date de publication de la demande: **24.07.2002**
(21) Numéro de dépôt: 02290101.1
(22) Date de dépôt: 16.01.2002
(51) Int. Cl.: C07D 233/54, C07D 401/06, C07D 213/38, A61K 31/415

(54) **Composés cycloheptène, leur procédé de préparation et les compositions pharmaceutiques qui les contiennent**

(30) Priorité: 18.01.2001 FR 0100641
(71) Demandeur: Les Laboratoires Servier S.A., 92200 Neuilly sur Seine (FR)
(72) Inventeur: Casara, Patrick, 78670 Villennes sur Seine (FR); Le Diguarher, Thierry, 92500 Rueil Malmaison (FR); Dorey, Gilbert, 78000 Versailles (FR); Hickman, John, 75017 Paris (FR); Pierre, Alain, 78580 Les Alluets le Roi (FR); Tucker, Gordon, 75017 Paris (FR); Guilbaud, Nicolas, 78170 La Celle Saint Cloud (FR)

(57) **Abrégé**

Composé de formule (I) : 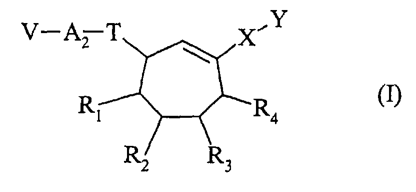 dans laquelle :
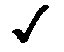 X représente une liaison ou un groupement alkylène, CO, S(O)ₙ, -S(O)ₙ-A₁-, -CO-A₁-, -A-S(O)ₙ-A'₁-, ou -A₁-CO-A'₁-,
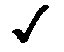 Y représente un groupement aryle, hétéroaryle, cycloalkyle ou hétérocycloalkyle, non substitués ou substitués,
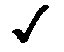 R₁, R₂, R₃ et R₄ représentent indépendamment un hydrogène ou un groupement aryle, hétéroaryle, cycloalkyle ou hétérocycloalkyle, non substitués ou substitués,
   ou bien R₁, R₂, R₃ et R₄, pris deux à deux, forment ensemble une liaison, ou forment un cycle benzénique fusionné ou un hétérocycle fusionné aromatique ou partiellement aromatique,
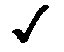 T représente un groupement -CH(R₅)-, -N(R₅)- ou -N(R₅)CO-,
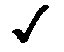 V représente un atome d'hydrogène ou un groupement aryle ou hétéroaryle non substitués ou substitués,
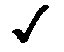 A₂ représente un groupement [C(R₆)(R'₆)]ₚ,
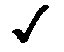 R₇ et R₈ sont tels que définis dans la description,

Médicaments.

## Description

La présente invention concerne de nouveaux composés cycloheptène, leur procédé de préparation et les compositions pharmaceutiques qui les contiennent.

Les composés de l'invention sont utiles comme inhibiteurs de famésyltransférase.

De nombreuses protéines subissent des altérations post-traductionnelles qui altèrent leur localisation et leur fonction. En particulier, des modifications de type lipidique permettent l'ancrage dans la membrane plasmique de certaines protéines inactives sous leur forme libre, une étape cruciale pour assurer leur fonction. C'est le cas de la prénylation *(Curr. Opin. Cell. Biol*., *4, 1992, 1008-1016*) qui est catalysée par plusieurs enzymes: la farnésyltransférase (FTase) et les deux géranylgéranyltransférases (GGTase-I et GGTase-II) qui accrochent un groupe prényl à 15 (*trans,trans*-farnésyl) ou 20 carbones (all-*trans*-géranylgéranyl) sur la partie carboxyterminale des protéines substrats (*J*. *Biol. Chem*., *271, 1996, 5289-5292 ; Curr. Opin. Struct. Biol., 7, 1997, 873-880*). La FTase catalyse ce transfert à partir de farnésyl pyrophosphate pour former un lien thioéther sur la cystéine de la séquence tétrapeptidique terminale consensus CA₁A₂X retrouvée sur les substrats protéines, où C désigne la cystéine, A₁ et A₂ un acide aminé aliphatique et X une sérine, une alanine ou une méthionine. La GGTase-I utilise du géranylgéranyl pyrophosphate comme substrat donneur pour effectuer un transfert similaire, mais cette fois la séquence consensus CAAX se termine par une leucine ou une phénylalanine. Ces deux enzymes hétérodimériques partagent une sous-unité alpha de 48 kDa, et possèdent deux chaînes bêta distinctes, quoique présentant 30% d'homologie de séquence en acides aminés. La GGTase-II agit sur des séquences terminales de types XXCC et XCXC et possède des sous-unités alpha et bêta différentes de celles des enzymes précédentes.

L'intérêt d'inhiber une de ces enzymes, la FTase a pour origine l'implication de l'oncogène prénylé Ras dans la progression tumorale (*Annu. Rev. Biochem*., *56, 1987, 779*-827). Les protéines Ras existent sous quatre formes majoritaires, Harvey ou H-Ras, N-Ras et Kirsten ou K-Ras A et B. Ces protéines sont exprimées sous une forme mutée dans au moins un quart des cancers avec une incidence encore plus forte pour certains types histologiques de tumeurs et selon la forme de Ras. Par exemple, des mutations de K-Ras B sont retrouvées dans 80 à 90% des carcinomes pancréatiques et 30 à 60% des cancers du côlon (*Int*. *J*. *Oncol.*, *7, 1995, 413-421*). De nombreuses données précliniques ont démontré le rôle de cet oncogène dans la progression tumorale, plus particulièrement dans les phénomènes de croissance cellulaire. Il s'agit d'un lien essentiel dans la transmission des signaux extracellulaires - comme ceux activés par les facteurs de croissance - vers diverses kinases cytosoliques puis vers le noyau, pour une intégration en terme de prolifération, mort cellulaire et survie cellulaire (*Cancer Met. Rev., 13, 1994, 67-89 ; Curr. Opin. Genetics & Develop*., *8, 1998, 49-54 ; J. Biol. Chem. 273, 1998, 19925-19928*), ou de régulation avec l'environnement tumoral - angiogenèse en particulier (*Cancer Res*., *55, 1995, 4575-4580*).

La recherche d'inhibiteurs de la FTase présente donc un intérêt majeur en oncologie (*Curr. Opin. Chem. Biol., 2, 1998, 40-48).* Comme 0.5% des protéines animales sont vraisemblablement prénylées et en majorité géranylgéranylées, des inhibiteurs spécifiques de la FTase par rapport aux GGTases, et plus particulièrement la GGTase-I, proche en structure de la FTase, sont d'un intérêt majeur. Les premiers travaux avec de tels inhibiteurs, des analogues peptidomimétiques de la séquence consensus de farnésylation, et les suivants avec des molécules issues de criblage de chimiothèques ont validé cette stratégie antitumorale lors d'expérimentations in vitro et chez l'animal (*Annu*. *Rev. Pharmacol*. *Toxicol*., *37, 1997, 143-166; Biochim. Biophys*. *Acta, 1423, 1999, C19-C30 ; Cancer Res., 58, 1998, 4947-4956*). Des fibroblastes spécialement transfectés avec le gène de la protéine H-Ras mutée et implantés chez l'animal développent une masse tumorale dont la croissance est diminuée en fonction de la dose d'inhibiteur de FTase reçue par l'animal. Pour des animaux transgéniques qui expriment une forme mutée de H-Ras sous le contrôle d'un promoteur approprié assurant l'apparition aléatoire de tumeurs salivaires ou mammaires spontanées, ces mêmes inhibiteurs entraînent la régression des tumeurs établies et bloquent l'apparition de nouvelles pendant la durée du traitement. Enfin, ces produits sont aussi actifs pour diminuer la croissance de xénogreffes humaines chez la souris, avec un effet possible d'augmentation de la survie, selon le modèle. La protéine Ras mutée n'est pas la seule cible indirecte de ces inhibiteurs dans la pathologie tumorale. L'étude de multiples modèles tumoraux a permis de constater une inhibition de la croissance tumorale indépendamment de la présence de protéines Ras mutées. Cet effet pourrait être en partie lié à une activité directe antiangiogénique et donc indépendante du profil oncogénique de la tumeur (*Eur. J*. *Cancer, 35, 1999, 1394-1401*). Cette observation renforce et élargit le potentiel d'utilisation antitumorale de cette classe d'inhibiteurs et l'absence d'effet secondaire rédhibitoire sur les fonctions cellulaires normales est aussi favorable à l'inhibition de la FTase dans toute pathologie associée à des mécanismes altérés ou amplifiés par une ou des protéines farnésylées. C'est notamment le cas, par exemple, en dehors du cancer, de la resténose après angioplastie ou chirurgie vasculaire, et de la neurofibromatose de type I (*Mol*. *Cell. Biol*., *17, 1997, 862-872*).

Les composés de l'invention présentent une structure originale et ont le pouvoir d'inhiber sélectivement la FTase vis à vis des GGTases. Ils seront donc utiles dans le traitement de toutes les pathologies associées à une signalisation intracellulaire passant par les protéines Ras ou d'autres protéines farnésylées, et dans les pathologies associées à une amplification de l'angiogenèse. Ainsi ils seront utiles dans le traitement du cancer, mais aussi de la resténose après angioplastie ou chirurgie vasculaire, et de la neurofibromatose de type I.

La présente invention concerne les composés de formule (I): 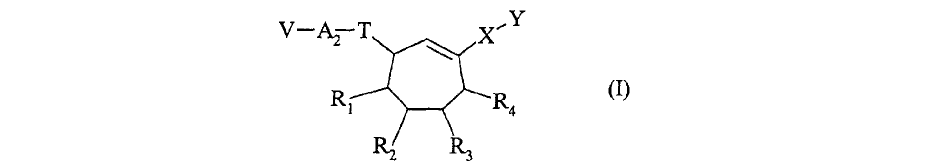 dans laquelle :
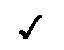 X représente une liaison ou un groupement choisi parmi alkylène, CO, S(O)ₙ, ∗-S(O)ₙ-A₁-, ∗-CO-A₁-, -A₁-S(O)ₙ-A'₁-, et -A₁-CO-A'₁- (dans lesquels A₁ et A'₁, identiques ou différents, représentent un groupement alkylène et n vaut 0, 1 ou 2), le symbole "∗" représentant le point de rattachement de ces groupements au cycloheptène,
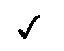 Y représente un groupement aryle, hétéroaryle, cycloalkyle ou hétérocycloalkyle, chacun de ces groupements étant non substitué ou substitué par un ou plusieurs, identiques ou différents, groupements R₈,
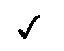 R₁, R₂, R₃ et R₄ représentent indépendamment un atome d'hydrogène ou un groupement aryle, hétéroaryle, cycloalkyle ou hétérocycloalkyle, chacun de ces groupements étant non substitué ou substitué par un groupement R₈,
   ou bien R₁, R₂, R₃ et R₄, pris deux à deux, forment ensemble une liaison,
   ou bien, R₁ et R₂, ou R₂ et R₃ ou R₃ et R₄ pris deux à deux avec les atomes de carbone auxquels ils sont liés, forment un cycle benzénique fusionné ou un hétérocycle fusionné aromatique ou partiellement hydrogéné, de 5 ou 6 chaînons contenant 1 ou 2 hétéroatomes choisis parmi azote, oxygène et soufre, sachant qu'un seul cycle peut être fusionné sur la structure à 7 chaînons,
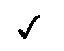 T représente un groupement -CH(R₅)-, -N(R₅)- ou ∗-N(R₅)CO- (où R₅ représente un atome d'hydrogène ou un groupement alkyle, aryle, hétéroaryle, arylalkyle ou hétéroarylalkyle, chacun de ces groupements étant non substitué ou substitué par un ou plusieurs, identiques ou différents, groupements R₇), le symbole "∗" représentant le point de rattachement de ce groupement au cycloheptène,
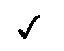 V représente un atome d'hydrogène ou un groupement aryle ou hétéroaryle, chacun de ces groupements étant non substitué ou substitué par un ou plusieurs, identiques ou différents, groupements R₇,
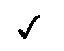 A₂ représente un groupement [C(R₆)(R'₆)]ₚ où p vaut 0, 1, 2, 3 ou 4 lorsque T représente un groupement -CH(R₅)- ou ∗-N(R₅)CO- ou p vaut 1, 2, 3 ou 4 lorsque T représente un groupement -N(R₅)-, et R₆ et R'₆, identiques ou différents, représentent un atome d'hydrogène ou un groupement alkyle, alkényle, alkynyle, aryle éventuellement substitué, hétéroaryle éventuellement substitué, hétérocycloalkyle éventuellement substitué, arylalkyle éventuellement substitué, hétéroarylalkyle éventuellement substitué, hétérocycloalkylalkyle éventuellement substitué, R₉ ou alkyle substitué par un groupement R₉ (où R₉ représente un groupement -OR₅, -N(R₅)(R'₅), -S(O)ₘR₅, -CON(R₅)(R'₅), -N(R₅)COR'₅, -N(R₅)SO₂R'₅, -SO₂N(R₅)(R'₅), -N(R₅)COO(R'₅), m étant égal à 0, 1 ou 2, et R'₅ peut prendre toutes les valeurs de R₅),
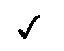 R₇ représente un atome d'halogène ou un groupement alkyle, alkoxy, hydroxy, mercapto, alkylthio, cyano, amino (éventuellement substitué par un ou deux groupements alkyle), nitro, carboxy, alkoxycarbonyle, aminocarbonyle (éventuellement substitué par un ou deux groupements alkyle), carbamoyle, aryle non substitué ou substitué, arylalkyle non substitué ou substitué, hétéroaryle non substitué ou substitué, hétéroarylalkyle non substitué ou substitué, cycloalkyle non substitué ou substitué, cycloalkylalkyle non substitué ou substitué, hétérocycloalkyle non substitué ou substitué ou hétérocycloalkylalkyle non substitué ou substitué,
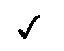 R₈ représente un atome d'halogène, ou un groupement oxo, hydroxy, cyano, nitro, carboxy, alkoxycarbonyl, perhalogénoalkyle ou un groupement -U-R₈₀, ou -A₈₀-U-R₈₀ (dans lesquels A₈₀ représente un groupement alkylène ; U représente une liaison, un atome d'oxygène, ou un groupement choisi parmi NH, S(O)ₘ, NHCO, CONH, SO₂NH, et NHSO₂, m étant égal à 0, 1, ou 2 ; et R₈₀ est un groupement choisi parmi alkyle, aryle, arylalkyle, hétéroaryle et hétéroarylalkyle),
étant entendu que :
- le terme alkyle désigne un groupement linéaire ou ramifié, contenant de 1 à 6 atomes de carbone,
- le terme alkylène désigne un groupement bivalent linéaire ou ramifié contenant de 1 à 6 atomes de carbone,
- le terme cycloalkyle désigne un groupement cyclique saturé contenant de 3 à 8 atomes de carbone,
- le terme hétérocycloalkyle désigne un groupement cyclique saturé ou partiellement insaturé de 5 à 7 chaînons contenant de 1 à 3 hétéroatomes choisis parmi azote, oxygène et soufre,
- le terme aryle désigne un groupement phényle ou naphtyle,
- le terme hétéroaryle désigne un groupement mono ou bicyclique, aromatique ou contenant au moins un cycle aromatique, de 5 à 11 chaînons, contenant de 1 à 5 hétéroatomes choisis parmi azote, oxygène et soufre,
- le terme substitué affecté aux expressions aryle, hétéroaryle, cycloalkyle, et hétérocycloalkyle signifie que ces groupements peuvent être substitués par un ou plusieurs groupements, identiques ou différents, choisis parmi cyano, alkylcarbonyle, aminocarbonyle (éventuellement substitué par un ou deux groupements alkyle), ou atomes d'halogène,
- le terme substitué affecté aux expressions arylalkyle, hétéroarylalkyle, cycloalkylalkyle, et hétérocycloalkylalkyle signifie que la partie cyclique de ces groupements peut être substituée par un ou plusieurs groupements, identiques ou différents, choisis parmi oxo, cyano, alkylcarbonyle, aminocarbonyle (éventuellement substitué par un ou deux groupements alkyle), ou atomes d'halogène,
leurs énantiomères, diastéréoisomères, ainsi que leurs sels d'addition à un acide ou à une base pharmaceutiquement acceptable.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, etc...

Parmi les bases pharmaceutiquement acceptables, on peut citer l'hydroxyde de sodium, l'hydroxyde de potassium, la triéthylamine, la tertbutylamine, etc...

Dans les composés de formule (I), X représente préférentiellement une liaison.

Des composés préférés de formule (I) sont ceux pour lesquels Y représente un groupement aryle (préférentiellement phényle éventuellement substitué par R₈).

Un aspect avantageux de l'invention concerne les composés de formule (I) pour lesquels R₁, R₂, R₃ et R₄ représentent chacun un atome d'hydrogène.

Plus particulièrement, l'invention concerne les composés de formule (I) pour lesquels T représente un groupement -N(R₅)- et encore plus préférentiellement un groupement -NH-.

Les groupements A₂ préférés sont le groupement méthylène, éthylène, (4-cyanophényl)méthylène, (4-chlorophényl)méthylène, (4-cyanobenzyl)méthylène ou (4-chlorobenzyl)méthylène.

Très avantageusement, V représente un groupement hétéroaryle comme par exemple les groupements pyridyle et 1*H*-imidazolyle, ces groupements étant préférentiellement substitués par un groupement arylalkyle éventuellement substitué comme par exemple le groupement *p*-cyanobenzyle ou *p*-chlorobenzyle.

Le groupement V-A₂-T- préféré de l'invention est le groupement [(4-cyanobenzyl)-1*H*-imidazol-5-yl]méthylamino.

Dans les composés préférés de formule (I) lorsque V est substitué par R₇, R₇ représente un groupement arylalkyle éventuellement substitué ou hétéroarylalkyle éventuellement substitué. Ces groupements sont avantageusement substitués par un atome d'halogène ou un groupement cyano.

Un aspect particulièrement avantageux de l'invention concerne les composés de formule (I) pour lesquels X représente une liaison, Y représente un groupement aryle éventuellement substitué par R₈, R₁, R₂, R₃ et R₄ représentent chacun un atome d'hydrogène, T représente un groupement -N(R₅)- et encore plus préférentiellement un groupement -NH-, A₂ représente un groupement -CH₂-, -CH₂-CH₂-, (4-cyanophényl)méthylène, (4-chlorophényl)méthylène, (4-cyanobenzyl)méthylène ou (4-chlorobenzyl)méthylène et V représente un groupement hétéroaryle comme par exemple les groupements pyridyle et 1*H*-imidazolyle éventuellement substitué par R₇.

Parmi les composés préférés de formule (I), on peut citer plus particulièrement : le 4-{[5-({[3-(2-méthylphényl)-2-cycloheptèn-1-yl]amino}méthyl)-1*H*-imidazol-1-yl] méthyl}benzonitrile, le 4-{[5-({[3-(3-méthylphényl)-2-cycloheptèn-1-yl]amino}méthyl)-1*H*-imidazol-1-yl]méthyl}benzonitrile et le 4-{[5-({[3-(3-chlorophényl)-2-cycloheptèn-1-yl]amino}méthyl)-1*H*-imidazol-1-yl]méthyl}benzonitrile.

La présente invention concerne également le procédé de préparation des composés de formule (I) caractérisé en ce que l'on utilise comme produit de départ un composé de formule (II) : 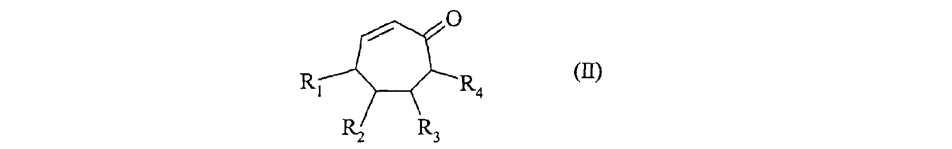 dans laquelle R₁, R₂, R₃ et R₄ sont tels que définis dans la formule (I),
dont la fonction carbonyle réagit avec un composé organométallique de formule Li-X-Y dans laquelle X et Y sont tels que définis dans la formule (I), pour conduire à un composé de formule (III) : 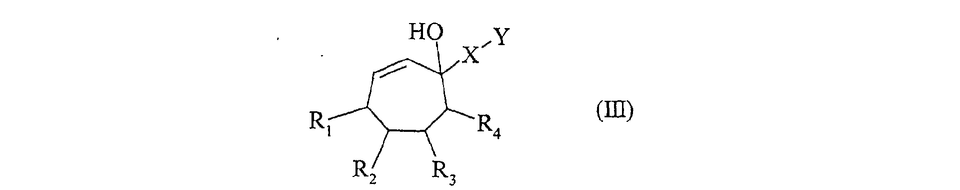 dans laquelle R₁, R₂, R₃, R₄, X et Y sont tels que définis précédemment,
qui subit une réaction d'isomérisation en milieu acide, pour conduire à un composé de formule (IV) : 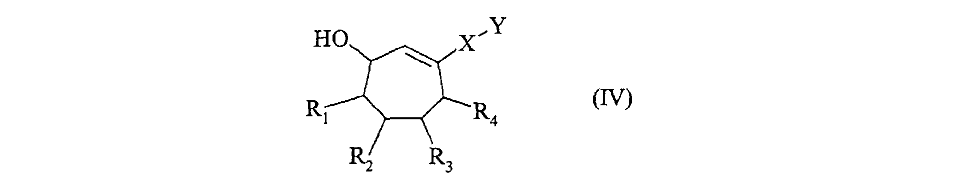 dans laquelle R₁, R₂, R₃, R₄, X et Y sont tels que définis précédemment,
composé de formule (IV), qui après transformation de la fonction hydroxyle en groupe partant, est soumis :
- soit à l'introduction d'une fonction réactive par action d'un dérivé silylé tel que Me₃SiCN, CH₂=CH-CH₂-SiMe₃ ou CH₂=C(Oalk)(OSiMe₃) où alk représente un groupement alkyle, puis à la condensation des groupements appropriés pour conduire à un composé de formule (I/a): 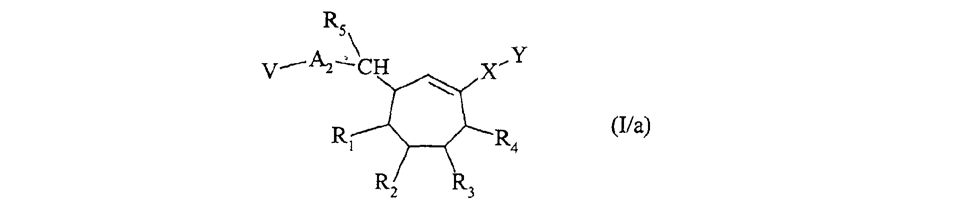 cas particulier des composés de formule (I) pour lequel R₁, R₂, R₃, R₄, R₅, A₂, V, X et Y sont tels que définis précédemment,
- soit à l'action de l'azidure de sodium, pour conduire, après hydrolyse en présence de triphénylphosphine, à une amine de formule (V) : 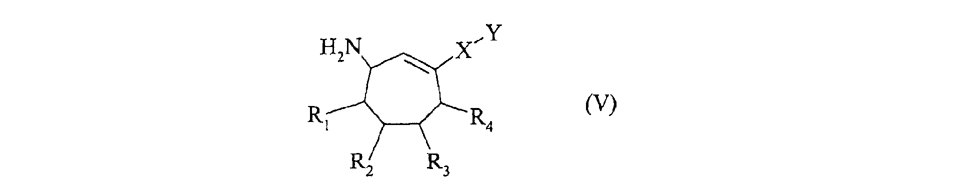
dans laquelle R₁, R₂, R₃, R₄, X et Y sont tels que définis précédemment,
composé de formule (V) qui est condensé sur un aldéhyde de formule : 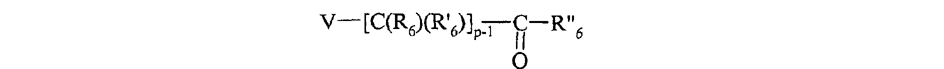 dans laquelle V est tel que défini précédemment, R₆ et R'₆ sont tels que définis dans la formule (I), R"₆ représente les mêmes atomes ou groupements que ceux définis par R₆ ou R'₆, et p est égal à 1, 2, 3 ou 4, pour conduire à un composé de formule (I/b): 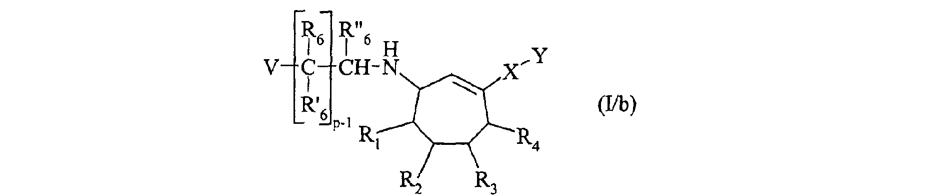 cas particulier des composés de formule (I) pour lequel R₁, R₂, R₃, R₄, R₆, R'₆, R"₆, V, X, Y et p sont tels que définis précédemment,
[certains composés de formule (I/b) pouvant par ailleurs être obtenus à partir du composé de formule (II') : 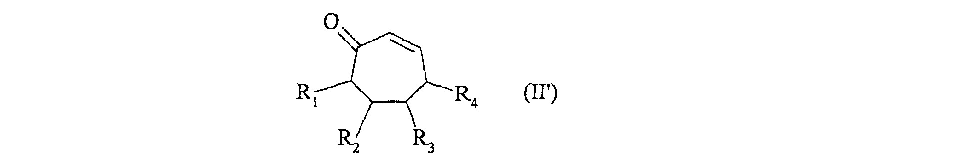 dans laquelle R₁, R₂, R₃ et R₄ sont tels que définis précédemment,
que l'on soumet, après formation de l'éther silylé correspondant, à l'action, en milieu basique fort, d'un composé Y-X'-CHO dans lequel Y est tel que défini précédemment et X' représente une liaison ou un groupement alkylène, pour conduire, après déprotection au composé de formule (III') : 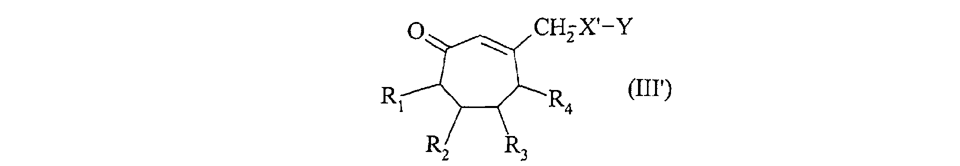 dans laquelle R₁, R₂, R₃, R₄, X' et Y sont tels que définis précédemment,
sur lequel on condense une amine de formule V-A₂-NH₂ dans laquelle V et A₂ sont tels que définis précédemment, pour obtenir le composé de formule (I/bₐ), cas particulier des composés de formule (I/b) : 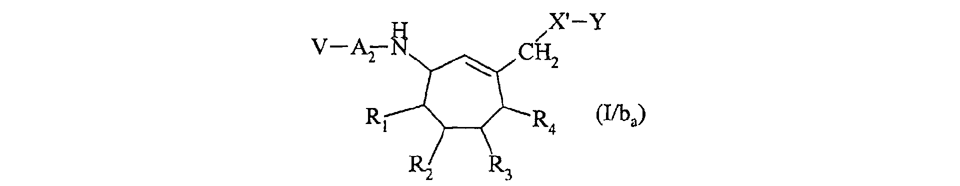 dans laquelle R₁, R₂, R₃, R₄, V, A₂, X' et Y sont tels que définis précédemment],
ou composé de formule (V) qui est soumis à une réaction d'acylation à l'aide d'un composé de formule V-A₂-CO-Hal dans laquelle V et A₂ sont tels que définis dans la formule (I) et Hal représente un atome d'halogène, ou à un couplage avec un acide carboxylique de formule V-A₂-COOH dans laquelle V et A₂ sont tels que définis dans la formule (I), pour conduire à un composé de formule (I'/b): 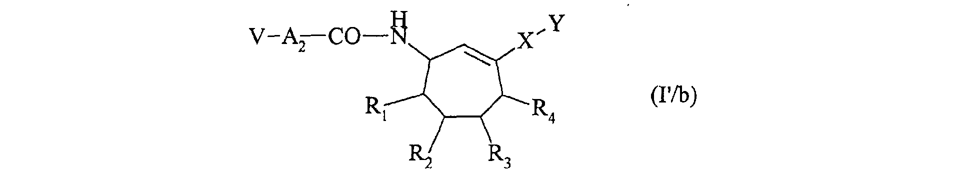 dans laquelle R₁, R₂, R₃, R₄, A₂, V, X et Y sont tels que définis précédemment,
composés (I/b) et (I'/b) qui peuvent être soumis au même type de condensation que précédemment, sur un aldéhyde de formule R"₅CHO, ou sur un halogénure d'acyle de formule R"₅-CO-Hal, ou encore sur un acide carboxylique de formule R"₅-COOH, dans lesquelles R"₅ peut prendre toutes les valeurs de R₅ à l'exception de l'atome d'hydrogène pour conduire à un composé de formule (I/c): 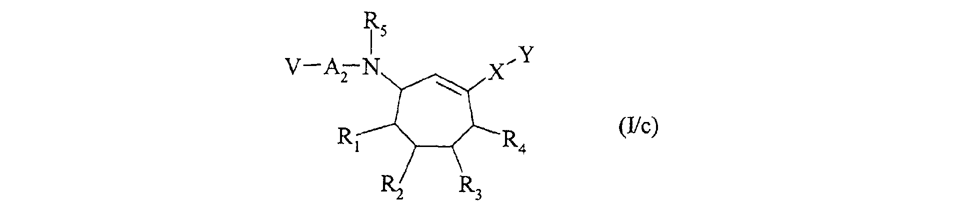 cas particulier des composés de formule (I) pour lequel R₁, R₂, R₃, R₄, R₅, A₂, V, X et Y sont tels que définis précédemment,
composés de formule (I/a), (I/b), (I'/b) et (I/c) formant la totalité des composés de formule (I),
- qui peuvent être, le cas échéant, purifiés selon une technique classique de purification,
- dont on sépare, le cas échéant, les isomères selon une technique classique de séparation,
- que l'on transforme, si on le souhaite, en ses leurs d'addition à un acide ou à une base pharmaceutiquement acceptable.

La présente invention a également pour objet les compositions pharmaceutiques renfermant comme principe actif au moins un composé de formule (I), seul ou en combinaison avec un ou plusieurs excipients ou véhicules inertes non toxiques, pharmaceutiquement acceptable.

Parmi les compositions pharmaceutiques selon l'invention, on pourra citer plus particulièrement celles qui conviennent pour l'administration orale, parentérale, nasale, transdermique, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,05 et 500 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles.

Les produits de départ utilisés sont des produits connus ou préparés selon des modes opératoires connus.

### PREPARATION 1 : 4-[(5-Formyl-1H-imidazol-1-yl)méthyl]benzonitrile

### Stade A : 4-{[5-(Hydroxyméthyl)-1H-imidazol-1-yl]méthyl}benzonitrile

A une solution de 4-(aminométhyl)benzonitrile (25 g/0,233 mole) dans 100 ml d'isopropanol, on ajoute successivement la dihydroxyacétone sous forme de dimère (23,35 g/0,129 mole) et le thioisocyanate de potassium (25,18 g/0,259 mole), puis le mélange est placé dans un bain de glace, et l'on ajoute goutte à goutte 20 ml d'acide acétique. Le milieu réactionnel est agité à température ambiante durant 48 heures. On obtient un précipité qui est filtré, lavé avec 50 ml d'isopropanol, puis deux fois avec 50 ml d'H₂O, puis séché. On obtient ainsi des cristaux qui sont directement engagés dans l'étape suivante de désulfùration : 13 g (0,059 mole) des cristaux obtenus précédemment sont placés dans 140 ml d'une solution diluée à 10% d'acide nitrique dans l'eau.A 0°C on ajoute très lentement 0,1 g de nitrite de sodium. On observe un fort dégagement d'un gaz brun et le mélange devient progressivement homogène. Le milieu réactionnel est ensuite agité à température ambiante durant 3 heures puis filtré et extrait une fois avec AcOEt. La phase aqueuse est ensuite basifiée avec une solution de soude 5N, puis extraite 2 fois avec AcOEt. La phase organique est lavée avec une solution saturée en NaCl, puis séchée sur MgSO₄ pour conduire, après évaporation sous vide, au produit du titre.

### Stade B : 4-[(5-Formyl-1H-imidazol-1-yl)méthyl]benzonitrile

A une solution de 4,6 g (24,9 mmoles) du composé obtenu au stade A dans 120 ml de DMSO, on ajoute successivement de la triéthylamine (13,8 ml/99,6 mmoles) puis le complexe SO₃-pyridine (9,89 g/62,25 mmoles) et le milieu réactionnel est agité à température ambiante durant 30 minutes. L'ensemble est ensuite placé à 0°C, hydrolysé avec H₂O, puis extrait plusieurs fois avec AcOEt. Les phases organiques sont regroupées, lavées avec une solution saturée en NaCl, séchées sur MgSO₄, et évaporées à sec pour conduire au produit du titre.

### PREPARATION 2 : 4-{[5-(Aminométhyl)-1H-imidazol-1-yl]méthyl}benzonitrile

A une solution du composé obtenu dans la Préparation 1 (1,05 g/4,97 mmoles) dans 50 ml de méthanol, on ajoute l'acétate d'ammonium 3,83 g (49,7 mmoles) et le NaBH₃CN (0,313g/4,97 mmoles) et l'ensemble est agité à température ambiante pendant 48 heures. Le milieu réactionnel est ensuite hydrolysé avec une solution de NaHCO₃ saturée, et extrait avec AcOEt. Les extraits organiques sont ensuite regroupés, séchés sur MgSO₄ et concentrés à sec. L'huile résiduelle est ensuite purifiée sur colonne de gel de silice (CH₂Cl₂, MeOH, NH₄OH, 98/1,5/0,5) pour conduire au produit du titre sous la forme d'une mousse blanche.

Les préparations 3 à 13 sont obtenues selon le même procédé que dans les Préparations 1 et 2 en remplaçant le 4-(aminométhyl)benzonitrile par le substrat approprié :

### PREPARATION 3 : 4-[1-(5-Formyl-1H-imidazol-1-yl)éthyl]benzonitrile

### PREPARATION 4 : 4-[(5-Formyl-1H-imidazol-1-yl)méthyl]cyclohexanecarbonitrile

### PREPARATION 5 : 1-[(1-Cyano-4-pipéridinyl)méthyl]-1H-imidazole-5-carbaldéhyde

### PREPARATION 6 : 1-(3-Phénylpropyl)-1H-imidazole-5-carbaldéhyde

### PREPARATION 7 : 1-(4-Fluorobenzyl)-1H-imidazole-5-carbaldéhyde

### PREPARATION 8 : 1-(4-Chlorobenzyl)-1H-imidazole-5-carbaldéhyde

### PREPARATION 9 : 1-(3-Chlorobenzyl)-1H-imidazole-5-carbaldéhyde

### PREPARATION 10 : 1-(4-Bromophényl)-1H-imidazole-5-carbaldéhyde

### PREPARATION 11 : 1-Benzyl-1H-imidazole-5-carbaldéhyde

### PREPARATION 12 : 4-{[5-(2-Oxoéthyl)-1H-imidazol-1-yl]méthyl}benzonitrile

### PREPARATION 13 : 1-(2-Phényléthyl)-1H-imidazole-5-carbaldéhyde

### PREPARATION 14 : 4-[(3-Formyl-4-pyridinyl)méthyl]benzonitrile

### Stade A : 4-(4-Cyanobenzyl)-3-formyl-1(4H)-pyridinecarboxylate de phényle

A une suspension de 1,44 g (0,022 mole) de zinc dans 20 ml de THF anhydre placée à -20°C, on ajoute goutte à goutte une solution de 4-bromobenzonitrile en solution dans 20 ml de THF. L'ensemble est agité à température ambiante durant 4 heures.

Parallèlement, on solubilise la 3-pyridine carboxaldéhyde (1,9 ml/0,02 mole) dans 20 ml de THF anhydre, puis on ajoute à 0°C le chlorofomate de phényl (2,5 ml/0,02 moles) en solution dans 10 ml de THF et le milieu réactionnel est agité à cette température pendant 1 heure. On observe la formation d'un précipité blanchâtre.

On transfert ensuite le bromozincique obtenu précédemment sur la pyridine protégée et l'ensemble est agité à 0°C 1,5 heures puis on laisse revenir progressivement à température ambiante et on laisse agiter 1,5 heure à cette température. On hydrolyse avec une solution de NH₄Cl saturée, on extrait avec l'AcOEt, on lave avec une solution de NaCl saturée, on sèche sur MgSO₄, puis on évapore à sec. On obtient une huile brune qui est purifiée par chromatographie sur gel de silice (heptane, AcOEt 10 %) pour obtenir le produit du titre.

### Stade B : 4-[(3-Formyl-4-pyridinyl)méthyl]benzonitrile

On solubilise le produit obtenu dans le stade A (2 g/0,0058 mole) dans 80 ml de décaline, puis on ajoute 0,336 g (0,010 mole) de soufre, et l'ensemble est chauffé à 140-150°C durant 24 heures. On filtre le milieu réactionnel, puis on le concentre. On obtient une huile brune qui est purifiée par chromatographie sur gel de silice (heptane, AcOEt 10%) pour conduire au produit du titre.

| *Microanalyse élémentaire :* | | | |
|---|---|---|---|
| | C% | H% | N% |
| Théorique | 75,65 | 4,53 | 12,60 |
| Trouvée | 75,50 | 4,32 | 12,50 |

### PREPARATION 15: 4-[(Imidazol-4-yl)-carbonyl]benzonitrile

### Stade A : N,N-Diméthyl-1H-imidazole-1-sulfonamide :

Ce dérivé est préparé selon le protocole décrit par D. J. Chadwick and R. I. Ngochindo, J. Chem. Soc., Perkin Trans., 481, 1984, à partir de 10,2 g (0,15 mole) d'imidazole. On obtient 20g d'une huile jaune translucide qui cristallise progressivement à température ambiante sous la forme d'un solide amorphe soit un rendement de 93%. IR 1177 et 1391 cm⁻¹, ν (NSO₂). RMN (CDCl₃) : 7.35, d, (1H); 7.25 d, (1H); 7.15 s, (1H) ; 2.31 s, (6H).

| *Microanalyse élémentaire :* | | | | |
|---|---|---|---|---|
| | C% | H% | N% | S% |
| Théorique | 34,28 | 5,18 | 23,98 | 18,30 |
| Trouvée | 34,71 | 5,53 | 23,02 | 18,47 |

### Stade B : 2-[tert-Butyl(diméthyl)silyl]-N,N-diméthyl-1H-imidazole-1-sulfonamide :

Ce dérivé est préparé selon le protocole décrit par J. W. Kim, S. M. Abdelaal and L. Bauer J. Heterocyclic Chem., 611 1995, par lithiation à -78°C du composé obtenu au stade B avec le n-Butyllithium (1,6M solution dans l'hexane), suivie de l'addition de TBDMSiCl. Après chromatographie sur gel de silice (acétate d'éthyle dans l'heptane) 2-[tert-butyl(diméthyl)silyl]-N,N-diméthyl-1*H*-imidazole-1-sulfonamide est isolé avec 80 % de rendement sous la forme d'une huile jaune translucide. IR 1176 et1386 cm⁻¹, ν (NSO₂). RMN (CDCl₃) : 7.35, d, (1H) ; 7.25 d, (1H) ; 2.85 , s, (6H) ; 1.0 s, (9H) ; 0.45, s, (6H).

### Stade C : 2-[tert-Butyl(diméthyl)silyl]-5-[(4-cyanophényl)(hydroxy)méthyl]-N,N-diméthyl-1H-imidazole-1-sulfonamide :

A une solution le composé obtenu au stade B (4,67g, 18 mmoles) dans 40 mL de THF anhydre placée à -78°C, on ajoute lentement une solution de n-Butyllithium (1,6M solution dans l'hexane), 12,5 mL (19,9 mmoles), puis l'ensemble est maintenu à cette température durant 1h30. On ajoute ensuite une solution de p-cyanobenzaldéhyde dans 20 mL de THF, 3,3g (25,1 mmoles). L'ensemble est agité à -78°C pendant 0,5h, puis hydrolysé avec une solution aqueuse de NaHCO₃ saturée. Lorsque le milieu réactionnel est à température ambiante, celui-ci est extrait avec AcOEt puis lavé avec une solution de NaCl saturée, séché sur MgSO₄ et concentré à sec.
Après purification sur gel de silice (heptane/AcOEt 3/1), on obtient le produit attendu 5,8g soit un rendement de 83 %. IR : 3449 ν (OH) ; 2230 ν(CN) ; 1609 ν(c=c) ; 1376 et 1146 ν cm⁻¹ (NSO₂). RMN (CDCl₃) : 7.7, d, (2H) ; 7.6 d, (2H) ;6.65 s (1H) ; 6.15 s (1H) ; 3.35 m (1H, OH) ; 2.85 , s, (6H) ; 1.0 s, (9H) ; 0.45, s, (6H).

### Stade D : 5-[(4-Cyanophényl)(hydroxy)méthyl]-N,N-diméthyl-1H-imidazole-1-sulfonamide

On solubilise le composé obtenu au stade C (4g, 9,5 mmoles) dans 40 mL de THF. On ajoute ensuite un mélange ACOH/H₂O (7:3) 40 mL et l'ensemble est agité à température ambiante pendant 2h. Le milieu réactionnel est ensuite hydrolysé dans un mélange de glace et d' H₂O, extrait avec AcOEt et lavé avec une solution de NaHCO₃ saturée, puis avec une solution de NaCl saturée, séché sur MgSO₄ et concentré à sec. Le solide résiduel est ensuite trituré dans l'heptane, le produit attendu est obtenu sous la forme de cristaux blancs 2,57g soit un rendement de 89%. IR : 3200 2700 ν(OH) ; 2230 ν(CN) ; 1390 et 1152 ν cm⁻¹(NSO₂). RMN (CDCl₃) : 7.9, s, (1H); 7.75 et 7.55 2d, (4H) ;6.55 s (1H); 6.15 d (1H) ; 3.25 d (1H, OH) ; 3.0 , s, (6H).

| *Microanalyse élémentaire :* | | | | |
|---|---|---|---|---|
| | C% | H% | N% | S% |
| Théorique | 50,97 | 4,61 | 18,29 | 10,47 |
| Trouvée | 51,58 | 4,76 | 18,05 | 10,12 |

### Stade E : 4-(1H-Imidazol-5-ylcarbonyl)benzonitrile:

4-(1*H*-imidazol-5-ylcarbonyl)benzonitrile est obtenu selon la méthode décrite par F. Effenberger ; M. Roos ; R. Ahmad ; et A. Krebs ; Chem. Ber. ; 124 (7) ; 1639-1650 ; 1991, à partir du composé obtenu au stade précédent par oxydation avec l'anhydride chromique dans l'acide acétique à reflux.

### PREPARATION 16: 4-[(1-Méthyl-1H-imidazol-5-yl)carbonyl]benzonitrile

### Stade A : 4-[(1-trityl-1H-imidazol-5-yl)carbonyl]benzonitrile :

Le dérivé obtenu au stade E de la préparation 15 est solubilisé dans le DMF et on ajoute successivement 2 équivalents molaire de Et₃N et 1,1 équivalent molaire de chlorure de triphénylméthyle. L'ensemble est agité à température ambiante 96h. Le milieu réactionnel est ensuite hydrolysé dans un mélange H₂O avec de la glace, et est extrait avec AcOEt. Après lavage, avec une solution diluée HCl à 1N, puis avec une solution de NaHCO₃ saturée, et finalement une solution de NaCI saturée, le milieu réactionnel est concentré à sec et purifié sur gel de silice (Heptane, AcOEt). On obtient le 4-[(1-trityl-1*H*-imidazol-5-yl)carbonyl]benzonitrile sous la forme d'un dérivé cristallin avec un rendement de 78 %.

### Stade B : 4-[(1-Méthyl-1H-imidazol-5-yl)carbonyl]benzonitrile :

Ce dérivé est obtenu à partir du composé préparé à l'étape précédente en utilisant la méthode décrite par I. M. Bell et all J . Med. Chem. ; 44 ; 2933-2949 2001, en 2 étapes en remplaçant le bromure de benzyle par l'iodure de méthyle comme agent alkylant.
Une étape de purification sur gel de silice permet d'isoler le 4-[(1-méthyl-1*H*-imidazol-5-yl)carbonyl]benzonitrile.

### PREPARATION 17: 4-[2-(1H-imidazol-5-yl)-2-oxoéthyl]benzonitrile

Ce dérivé est obtenu selon le même procédé que pour la préparation 15, en utilisant au stade C le 4-(cyanophényl)acétaldéhyde comme agent alkylant à la place du p-cyanobenzaldéhyde.

### PREPARATION 18: 4-[2-(1-Méthyl-1H-imidazol-5-yl)-2-oxoéthyl]benzonitrile

Ce dérivé est obtenu selon le même procédé que pour la préparation 16, en utilisant le composé obtenu dans la préparation 17.

### PREPARATION 19: (4-Chlorophényl)(1H-imidazol-5-yl)méthanone

Ce dérivé est obtenu selon le même procédé que dans la préparation 15, en utilisant au stade C le 4-(chlorophényl)benzaldéhyde à la place du p-cyanobenzaldéhyde comme agent alkylant.

### PREPARATION 20: (4-Chlorophényl)(1-méthyl-1H-imidazol-5-yl)méthanone

Ce dérivé est obtenu selon le même procédé que la préparation 16, en utilisant le composé obtenu dans la préparation 19.

### PREPARATION 21: 2-(4-Chlorophényl)-1-(1H-imidazol-5-yl)éthanone

Ce dérivé est obtenu selon le même procédé que la préparation 15, en utilisant au stade C le 4-(chlorophényl)acétaldéhyde à la place du p-cyanobenzaldéhyde comme agent alkylant.

### PREPARATION 22: 2-(4-Chlorophényl)-1-(1-méthyl-1H-imidazol-5-yl)éthanone

Ce dérivé est obtenu selon le même procédé que la préparation 16, décrit précédemment, en utilisant le composé obtenu dans la préparation 21.

### EXEMPLE 1 : 4-{[5-({[3-(2-Méthylphényl)-2-cycloheptèn-1-yl]amino}méthyl)-1H-imidazol-1-yl]méthyl}benzonitrile

### Stade A : 1-(2-Méthylphényl)-2-cycloheptèn-1-ol

Ce dérivé est obtenu en adoptant la méthode décrite dans *Tetrahedron* Vol 52, n°9, pp 3107-3116, 1996.
A une solution d'α-bromotoluène 3,25 ml (26,9 mmoles) dans un mélange THF/Et₂O (50/25 ml) placée à -78°C , on ajoute lentement 16,9 ml (26,9 mmoles) d'une solution de nBuLi (1,6 M/hexane). L'ensemble est agité à cette température durant 30 minutes, puis on ajoute la cycloheptènone (3 ml/26,9 mmoles), et le mélange est agité à cette température 1 heure, puis progressivement à température ambiante durant la nuit. Le milieu réactionnel est ensuite hydrolysé dans une solution de NaHCO₃ saturée, et extrait avec Et₂O. Après lavage avec une solution saturée en NaCl, séchage sur MgSO₄, et évaporation à sec, on obtient une huile brune qui est purifiée par chromatographie sur gel de silice (heptane, AcOEt 2 %) pour conduire au produit du titre sous la forme d'une huile incolore.

| *Microanalyse élémentaire* | | |
|---|---|---|
| | C% | H% |
| Théorique | 83,12 | 8,97 |
| Trouvée | 83,26 | 8,75 |

### Stade B : 3-(2-Méthylphényl)-2-cycloheptèn-1-ol

A une solution du composé obtenu au stade A (3,7 g/18,29 mmoles) dans 100 ml de dioxane, on ajoute goutte à goutte une solution diluée à 1% de H₂SO₄ concentré dans H₂O. (100 ml). L'ensemble est agité ensuite à température ambiante 2 heures. Le milieu réactionnel est extrait avec Et₂O, lavé avec une solution de NaHCO₃ saturée, puis une solution de NaCl saturée jusqu'à neutralité. Après séchage sur MgSO₄ et évaporation à sec, on obtient une huile qui est purifiée par chromatographie sur gel de silice (heptane, AcOEt, 4/1) pour conduire au produit du titre sous la forme d'une huile incolore.

| *Microanalyse élémentaire* | | |
|---|---|---|
| | C% | H% |
| Théorique | 83,12 | 8,97 |
| Trouvée | 83,70 | 8,77 |

### Stade C : 3-(2-Méthylphényl)-2-cycloheptèn-1-yl acétate

A une solution du composé obtenu au stade B (6,2 g/0,03 mole) dans 100 ml de CH₂Cl₂ on ajoute successivement la pyridine (4,9 ml/0,06 mole), le DMAP (1,83 g/0,015 mole), puis l'anhydride acétique (5,7 ml/0,06 mole) goutte à goutte. L'ensemble est ensuite agité à température ambiante durant 24 heures. Le milieu réactionnel est ensuite concentré à sec, repris avec AcOEt, puis lavé avec une solution diluée en HCl (1N), une solution de NaHCO₃ saturée, puis avec une solution de NaCl saturée; séché sur MgSO₄, puis concentré à sec. L'huile résiduelle jaunâtre est ensuite purifiée par chromatographie sur gel de silice pour conduire au preoduit du titre sous la forme de cristaux blancs.

### Stade D : 3-Azido-1-(2-méthylphényl)-1-cycloheptène

A une solution du composé obtenu au stade C (3,52 g/14,65 mmoles) dans 50 ml de 1,2-dichloroéthane, on ajoute goutte à goutte le triméthylsilyl azide (TMSN₃), puis 0,326 g (1,46 mmoles) de perchlorate de magnésium (Mg(ClO₄)₂) et l'ensemble est agité à température ambiante une nuit. On hydrolyse ensuite avec H₂O puis on extrait avec AcOEt; les phases organiques sont alors regroupées, lavées avec une solution de NaCl saturée, séchées sur MgSO₄, concentrées à sec. L'huile jaune ainsi obtenue est chromatographiée sur colonne de gel de silice (cyclohexane, AcOEt 2 %) pour conduire au produit du titre sous la forme d'une huile translucide.

### Stade E : 3-(2-Méthylphényl)-2-cycloheptèn-1-ylamine

A une solution du composé obtenu au stade D (2,4 g/10,73 mmoles) dans 60 ml de THF, on ajoute 2,5 ml d'H₂O puis 4,23 g (16,1mmoles) de triphénylphosphine PPh₃ puis le milieu réactionnel est agité à température ambiante durant une nuit. L'ensemble est ensuite concentré à sec puis chromatographié sur gel de silice (CH₂Cl₂, MeOH 20%, NH₄OH 2 %) pour conduire au produit du titre sous la forme dune huile jaunâtre.

| *Microanalyse élémentaire* | | | |
|---|---|---|---|
| | C% | H% | N% |
| Théorique | 83,53 | 9,51 | 6,96 |
| Trouvée | 82,71 | 9,32 | 7,09 |

### Stade F : 4-{[5-({[3-(2-Méthylphényl)-2-cycloheptèn-1-yl]amino}méthyl)-1H-imidazol-1-yl]méthyl}benzonitrile

A une solution du composé obtenu dans le stade E (0,746 g/3,7 mmoles) dans 30 ml de C₂H₄Cl₂ on ajoute 0,82 g (3,88 mmoles) du composé obtenu dans la Préparation 1, puis le NaHB(OAc)₃ (1,18 g/5,55 mmoles) et l'ensemble est agité à température ambiante durant 48 heures. Le milieu réactionnel est ensuite hydrolysé dans l'eau, extrait avec AcOEt puis lavé jusqu'à neutralité. Après séchage sur MgSO₄ et évaporation à sec, l'huile résiduelle est alors purifiée par chromatographie sur gel de silice (CH₂Cl₂/MeOH/NH₄OH : 97,5/2/0,5). On obtient le produit du titre sous la forme d'une résine translucide qui est directement solubilisée dans 40 ml d'un mélange CH₃CN/H₂O 50/50.
Puis une salification est réalisée avec de l'acide fumarique pour conduire au bis-fumarate correspondant qui est lyophilisé :
Point de fusion : 60-63°C

| *Microanalyse élémentaire* | | | |
|---|---|---|---|
| | C% | H% | N% |
| Théorique | 64,96 | 5,77 | 8,91 |
| Trouvée | 65,24 | 5,69 | 8,91 |

### EXEMPLE 2 : 4-[(5-{[(3-Phényl-2-cycloheptèn-1-yl)amino]méthyl}-1H-imidazol-1-yl)méthyl]benzonitrile

On procède comme dans l'Exemple 1 en remplaçant au stade A le 1-bromo-2-méthylbenzène par le bromobenzène.
Une salification est réalisée avec de l'acide fumarique pour conduire au fumarate correspondant :

| *Microanalyse élémentaire* | | | |
|---|---|---|---|
| | C% | H% | N% |
| Théorique | 69,22 | 6,01 | 10,98 |
| Trouvée | 69,14 | 6,14 | 10,25 |

### EXEMPLE 3 : 4-{[5-({[3-(2-Méthylhenzyl)-2-cycloheptén-1-yl]amine}méthyl)-1H-imidazol-1-yl]méthyl}benzonitrile

### Stade A : tert-Butyl(diméthyl){[3-(2-méthylbenzylidène)-1-cycloheptèn-1-yl]oxy}silane

A une solution de cycloheptènone (71,7 mmoles) dans 220 ml de THF anhydre on ajoute successivement la triphényle phosphine (72,6 mmoles) puis goutte à goutte le *tert* butyldiméthylsilyltriflate (72,3 mmoles) et l'ensemble est agité à température ambiante pendant 1 heure 30 minutes. On se place ensuite à -78°C et on ajoute lentement une solution de nBuLi /Hexane (72 mmoles) puis après 30 minutes on ajoute le 2-méthylbenzaldéhyde (72,03 mmoles). L'ensemble est agité à cette température pendant 30 minutes, puis progressivement à température ambiante. Après 2 heures 30 minutes le milieu réactionnel est précipité dans 2,5 1 d'éther de pétrole, filtré et le filtrat est concentré à sec. Le produit du titre est purifié par chromatographie sur gel de silice (heptane 100 %).

### Stade B : (2-Méthylbenzyl)-2-cycloheptèn-1-one

A une solution du composé obtenu au stade A (1,34 mmoles) dans 15 ml de THF anhydre placée à 0°C, on ajoute via une seringue une solution de fluorure de *n-tétra-n*-butyl ammonium (1M) dans le THF (1,35 mmoles) et l'ensemble est agité à cette température pendant 1 heure 30 minutes. Puis le milieu réactionnel est concentré à sec et déposé directement sur une colonne de gel de silice (heptane/AcOEt 9/1) pour conduire au produit du titre.

### Stade C : 4-{[5-({[3-(2-Méthylbenzyl)-2-cycloheptèn-1-yl]amino}méthyl)-1H-imidazol-1-yl]méthyl}benzonitrile

Le composé du titre est obtenu par couplage entre le composé obtenu dans la Préparation 2 (2 mmoles) et le composé obtenu au stade B (2 mmoles) en présence de Zn(BH₄)₂ (2 mmoles) selon la méthode décrite dans *J. Org. Chem.* 1998, 63, 370. Le produit du titre est obtenu pur après purification sur gel de silice (CH₂Cl₂, MeOH, NH₄OH, 95/4/1) sous la forme d' une gomme translucide qui est transformée en sel de fumarate.

### EXEMPLE 4 : 4-[(5-{[(3-Benzyl-2-cycloheptèn-1-yl)amino]méthyl}-1H-imidazol-1-yl)méthyl]benzonitrile

On procède comme dans l'Exemple 3 en remplaçant au stade A le 2-méthylbenzaldéhyde par le benzaldéhyde.

### EXEMPLE 5 : 4-{[5-({[3-(3-Méthylphényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-3-méthylbenzène.
Une salification est réalisée avec de l'acide fumarique pour conduire au fumarate correspondant :

| *Microanalyse élémentaire* | | | |
|---|---|---|---|
| | C% | H% | N% |
| Théorique | 70,29 | 6,29 | 10,93 |
| Trouvée | 70,39 | 6,06 | 10,90 |

### EXEMPLE 6 : 4-{[5-({[3-(3-Méthylbenzyl)-2-cycloheptén-1-yl]amino}méthyl)-1H-imidazol-1-yl]méthyl}benzonitrile

On procède comme dans l'Exemple 3 en remplaçant au stade A le 2-méthylbenzaldéhyde par le 3-méthylbenzaldéhyde.

### EXEMPLE 7 : 4-{[5-({[3-(4-Méthylbenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 4-méthylbenzaldéhyde.

### EXEMPLE 8 : 4-{[5-({[3-(4-Méthylphényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-4-méthylbenzène.
Une salification est réalisée avec de l'acide fumarique pour conduire au fumarate correspondant :

| *Microanalyse élémentaire* | | | |
|---|---|---|---|
| | C% | H% | N% |
| Théorique | 70,29 | 6,29 | 10,93 |
| Trouvée | 70,31 | 6,08 | 10,88 |

### EXEMPLE 9 : 4-{[5-({[3-(3-Chlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-3-chlorobenzène.

| *Microanalyse élémentaire* | | | |
|---|---|---|---|
| | C% | H% | N% |
| Théorique | 61,30 | 5,56 | 11,44 |
| Trouvée | 61,77 | 5,58 | 11,26 |

### EXEMPLE 10 : 4-{[5-({[3-(3-Chlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 3-chlorobenzaldéhyde.

### EXEMPLE 11 : 4-{[5-({[3-(4-Chlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-4-chlorobenzène.

### EXEMPLE 12 : 4-{[5-({[3-(4-Chlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 4-chlorobenzaldéhyde.

### EXEMPLE 13: 4-{[5-({[3-(2-Chlorophényl)-2-cycloheptén-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-2-chlorobenzène.

### EXEMPLE 14 : 4-{[5-({[3-(2-Chlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 2-chlorobenzaldéhyde.

### EXEMPLE 15 : 4-{[5-({[3-(2,3-Dichlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-2,3-dichlorobenzène.

### EXEMPLE 16 : 4-{[5-({[3-(2,3-Dichlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 2,3-dichlorobenzaldéhyde.

### EXEMPLE 17 : 4-{[5-({[3-(2,4-Dichlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 2,4-dichlorobenzaldéhyde.

### EXEMPLE 18 : 4-{[5-({[3-(2,4-Dichlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-2,4-dichlorobenzène.

### EXEMPLE 19 : 4-{[5-({[3-(2,5-Dichlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-2,5-dichlorobenzène.

### EXEMPLE 20 : 4-{[5-({[3-(2,5-Dichlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 2,5-dichlorobenzaldéhyde.

### EXEMPLE 21 : 4-{[5-({[3-(3,5-Dichlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 3,5-dichlorobenzaldéhyde.

### EXEMPLE 22 : 4-{[5-({[3-(3,5-Dichlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-3,5-dichlorobenzène.

### EXEMPLE 23 : 4-{[5-({[3-(3,4-Dichlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 3,4-dichlorobenzaldéhyde.

### EXEMPLE 24 : 4-{[5-({[3-(3,4-Dichlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-3,4-dichlorobenzène.

### EXEMPLE 25 : 4-{[5-({[3-(2,3-Dichlorophényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-2,3-dichlorobenzène.

### EXEMPLE 26 : 4-{[5-({[3-(2,3-Dichlorobenzyl)-2-cycloheptèn-1-yl]amino}méthyl)-1H-imidazol-1-yl]méthyl}benzonitrile

On procède comme dans l'Exemple 3 en remplaçant au stade A le 2-méthylbenzaldéhyde par le 2,3-dichlorobenzaldéhyde.

### EXEMPLE 27 : 4-{[5-({[3-(4-Chloro-2-méthylphényl)-2-cycloheptèn-1-yl]amino} méthyl)-1H-imidazol-1-yl]méthyl}benzonitrile

On procède comme dans l'Exemple 1 en remplaçant au stade A le 1-bromo-2-méthylbenzène par le 1-bromo-4-chloro-2-méthylbenzène.

### EXEMPLE 28 : 4-{[5-({[3-(4-Fluoro-2-méthylphényl)-2-cycloheptèn-1-yl]amino} méthyl)-1H-imidazol-1-yl]méthyl}benzonitrile

On procède comme dans l'Exemple 1 en remplaçant au stade A le 1-bromo-2-méthylbenzène par le 1-bromo-4-fluoro-2-méthylbenzène.

### EXEMPLE 29 : 4-{[5-({[3-(2,4-Diméthylphényl)-2-cycloheptèn-1-yl]amino}méthyl)-1H-imidazol-1-yl]méthyl}benzonitrile

On procède comme dans l'Exemple 1 en remplaçant au stade A le 1-bromo-2-méthylbenzène par le 1-bromo-2,4-diméthylbenzène.

### EXEMPLE 30 : 4-{1-[5-({[3-(2-Méthylphenyl)-2-cycloheptèn-1-yl]amino}méthyl)-1H-imidazol-1-yl]éthyl}benzonitrile

On procède comme dans l'Exemple 1 en remplaçant au stade F le composé obtenu dans la Préparation 1 par le composé obtenu dans la Préparation 3.

### EXEMPLE 31 : 4-{[3-({[3-(2-Méthylphényl)-2-cycloheptèn-1-yl]amino}méthyl)-4-pyridinyl]méthyl}benzonitrile

On procède comme dans l'Exemple 1 en remplaçant au stade F le composé obtenu dans la Préparation 1 par le composé obtenu dans la Préparation 14.
Une salification est réalisée avec de l'acide fumarique pour conduire au bis-fumarate correspondant :

| *Microanalyse élémentaire* | | | |
|---|---|---|---|
| | C% | H% | N% |
| Théorique | 67,59 | 5,83 | 6,57 |
| Trouvée | 67,04 | 5,72 | 6,07 |

### EXEMPLE 32 : 4-{[5-({[3-(2-Méthylphényl)-2-cycloheptèn-1-yl]amino}méthyl)-1H-imidazol-1-yl]méthyl}cyclohexanecarbonitrile

On procède comme dans l'Exemple 1 en remplaçant au stade F le composé obtenu dans la Préparation 1 par le composé obtenu dans la Préparation 4.

### EXEMPLE 33 : N-({1-[(1-Cyano-4-pipéridinyl)méthyl]-1H-imidazol-5-yl}méthyl)-N-[3-(2-méthylphényl)-2-cycloheptèn-1-yl]amine

On procède comme dans l'Exemple 1 en remplaçant au stade F le composé obtenu dans la Préparation 1 par le composé obtenu dans la Préparation 5.

### EXEMPLE 34 : 4-{[5-({[3-(3-Méthylphényl)-2-cycloheptèn-1-yl]amino}méthyl)-1H-imidazo]-1-yl]méthyl}cyclohexanecarbonitrile

On procède comme dans l'Exemple 1 en remplaçant au stade A le 1-bromo-2-méthylbenzène par le 1-bromo-3-méthylbenzène et en remplaçant au stade F le composé obtenu dans la Préparation 1 par le composé obtenu dans la Préparation 4.

### EXEMPLE 35 : N-({1-[(1-Cyano-4-pipéridinyl)méthyl]-1H-imidazol-5-yl}méthyl)-N- [3-(3-méthylphényl)-2-cycloheptèn-1-yl]amine

On procède comme dans l'Exemple 1 en remplaçant au stade A le 1-bromo-2-méthylbenzène par le 1-bromo-3-méthylbenzène et en remplaçant au stade F le composé obtenu dans la Préparation 1 par le composé obtenu dans la Préparation 5.

### EXEMPLE 36 : 4-{[5-({[3-(2-Chlorophényl)-2-cycloheptèn-1-yl]amino}méthyl)-1H-imidazol-1-yl]méthyl}cyclohexanecarbonitrile

On procède comme dans l'Exemple 1 en remplaçant au stade A le 1-bromo-2-méthylbenzène par le 1-bromo-2-chlorobenzène et en remplaçant au stade F le composé obtenu dans la Préparation 1 par le composé obtenu dans la Préparation 4.

### EXEMPLE 37 : 4-{[5-({[3-(3-Chlorophényl)-2-cycloheptèn-1-yl]amino}méthyl)-1H-imidazol-1-yl]méthyl}cyclohexanecarbonitrile

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 4.

### EXEMPLE 38 : N-({1-[(1-Cyano-4-pipéridinyl)méthyl]-1H-imidazol-5-yl}méthyl)-N-[3-(2-chlorophényl)-2-cycloheptèn-1-yl]amine

On procède comme dans l'Exemple 1 en remplaçant au stade A le 1-bromo-2-méthylbenzène par le 1-bromo-2-chlorobenzène et en remplaçant au stade F le composé obtenu dans la Préparation 1 par le composé obtenu dans la Préparation 5.

### EXEMPLE 39 : N-({1-[(1-Cyano-4-pipéridinyl)méthyl]-1H-imidazol-5-yl}méthyl)-N-[3-(3-chlorophényl)-2-cycloheptèn-1-yl]amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 5.

### EXEMPLE 40 : 4-[(5-{[(3-Mésityl-2-cycloheptèn-1-yl)amino]méthyl}-1H-imidazol-1-yl)méthyl]benzonitrile

On procède comme dans l'Exemple 1 en remplaçant au stade A le 1-bromo-2-méthylbenzène par le 1-bromo-2,4,6-triméthylbenzène.
Une salification est réalisée avec de l'acide fumarique pour conduire au bis-fumarate correspondant :

| *Microanalyse élémentaire* | | | |
|---|---|---|---|
| | C% | H% | N% |
| Théorique | 65,84 | 6,14 | 8,53 |
| Trouvée | 65,48 | 6,07 | 8,48 |

### EXEMPLE 41 : 4-({5-[({3-[(Phénylthio)méthyl]-2-cycloheptèn-1-yl}amino)méthyl]-1H-imidazol-1-yl}méthyl)benzonitrile

On procède comme dans l'Exemple 1 en remplaçant au stade A le 1-bromo-2-méthylbenzène par le [(bromométhyl)thio]benzène.
Une salification est réalisée avec de l'acide fumarique pour conduire au bis-fumarate correspondant :

| *Microanalyse élémentaire* | | | | |
|---|---|---|---|---|
| | C% | H% | N% | S% |
| Théorique | 61,81 | 5,49 | 8,48 | 4,85 |
| Trouvée | 61,99 | 5,76 | 8,45 | 4,82 |

### EXEMPLE 42 : N-{3-[(Phénylthio)méthyl]-2-cycloheptèn-1-yl}-N-(3-pyridinyl-méthyl)amine

On procède comme dans l'Exemple 1 en remplaçant au stade A le 1-bromo-2-méthylbenzène par le [(bromométhyl)thio]benzène et en remplaçant au stade F le composé obtenu dans la Préparation 1 par le nicotinaldehyde.
Une salification est réalisée avec de l'acide fumarique pour conduire au bis-fumarate correspondant :

| *Microanalyse élémentaire* | | | | |
|---|---|---|---|---|
| | C% | H% | N% | S% |
| Théorique | 60,42 | 5,79 | 5,03 | 5,76 |
| Trouvée | 60,51 | 5,85 | 4,89 | 5,60 |

### EXEMPLE 43 : 4-{[{3-[(Phénylthio)méthyl]-2-cycloheptèn-1-yl}(3-pyridinylméthyl) amino]méthyl}benzonitrile

On procède comme au stade F de l'Exemple 1 à partir du composé obtenu dans l'Exemple 42 en remplaçant le composé obtenu dans la Préparation 1 par le 4-formylbenzonitrile.
Une salification est réalisée avec de l'acide fumarique pour conduire au bis-fumarate correspondant :

| *Microanalyse élémentaire* | | | | |
|---|---|---|---|---|
| | C% | H% | N% | S% |
| Théorique | 65,19 | 5,63 | 6,48 | 4,94 |
| Trouvée | 65,35 | 5,83 | 6,58 | 4,92 |

### EXEMPLE 44 : N-[(1-Benzyl-1H-imidazol-5-yl)méthyl)-N-[3-(2-méthylphényl)-2-cycloheptèn-1-yl]amine

On procède comme dans l'Exemple 1 en remplaçant au stade F le composé obtenu dans la Préparation 1 par le composé obtenu dans la Préparation 11.

### EXEMPLE 45 : N-[3-(2-Méthylphényl)-2-cycloheptèn-1-yl]-N-{[1-(2-phényléthyl)-1H-imidazol-5-yl]méthyl}amine

On procède comme dans l'Exemple 1 en remplaçant au stade F le composé obtenu dans la Préparation 1 par le composé obtenu dans la Préparation 13.

### EXEMPLE 46 : N-[3-(2-Méthylphényl)-2-cycloheptèn-1-yl]-N-{[1-(3-phénylpropyl)-1H-imidazol-5-yl]méthyl}amine

On procède comme dans l'Exemple 1 en remplaçant au stade F le composé obtenu dans la Préparation 1 par le composé obtenu dans la Préparation 6.

### EXEMPLE 47 : N-{[1-(4-Chlorobenzyl)-1H-imidazol-5-yl]méthyl}-N-[3-(2-méthylphényl)-2-cycloheptèn-1-yl]amine

On procède comme dans l'Exemple 1 en remplaçant au stade F le composé obtenu dans la Préparation 1 par le composé obtenu dans la Préparation 8.

### EXEMPLE 48 : N-{[1-(3-Chlorobenzyl)-1H-imidazol-5-yl]méthyl}-N-[3-(2-méthylphényl)-2-cycloheptèn-1-yl]amine

On procède comme dans l'Exemple 1 en remplaçant au stade F le composé obtenu dans la Préparation 1 par le composé obtenu dans la Préparation 9.

### EXEMPLE 49 : N-{[1-(4-Bromobenzyl)-1H-imidazol-5-yl]méthyl}-N-[3-(2-méthylphényl)-2-cycloheptèn-1-yl]amine

On procède comme dans l'Exemple 1 en remplaçant au stade F le composé obtenu dans la Préparation 1 par le composé obtenu dans la Préparation 10.

### EXEMPLE 50 : N-[(1-Benzyl-1H-imidazol-5-yl)méthyl]-N-(3-phényl-2-cycloheptèn-1-yl)amine

On procède comme dans l'Exemple 1 en remplaçant au stade A le 1-bromo-2-méthylbenzène par le bromobenzène et en remplaçant au stade F le composé obtenu dans la Préparation 1 par le composé obtenu dans la Préparation 11.

### EXEMPLE 51 : N-[(1-Benzyl-1H-imidazol-5-yl)méthyl]-N-[3-(3-chlorophényl)-2-cycloheptèn-1-yl]amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 11.

### EXEMPLE 52 : N-{[1-(4-Fluorobenzyl)-1H-imidazol-5-yl]méthyl}-N-(3-phényl-2-cycloheptèn-1-yl)amine

On procède comme dans l'Exemple 1 en remplaçant au stade A le 1-bromo-2-méthylbenzène par le bromobenzène et en remplaçant au stade F le composé obtenu dans la Préparation 1 par le composé obtenu dans la Préparation 7.

### EXEMPLE 53 : N-{[1-(4-Chlorobenzyl)-1H-imidazol-5-yl]méthyl}-N-[3-(2-chlorophényl)-2-cycloheptèn-1-yl]amine

On procède comme dans l'Exemple 1 en remplaçant au stade A le 1-bromo-2-méthylbenzène par le 1-bromo-2-chlorobenzène et en remplaçant au stade F le composé obtenu dans la Préparation 1 par le composé obtenu dans la Préparation 8.

### EXEMPLE 54 : N-{[1-(3-Chlorobenzyl)-1H-imidazol-5-yl]méthyl}-N-[3-(3-chlorophényl)-2-cycloheptèn-1-yl]amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 9.

### EXEMPLE 55 : 4-{[3-({[3-(3-Chlorophényl)-2-cycloheptèn-1-yl]amino}méthyl)-4-pyridinyl]méthyl}benzonitrile

On procède comme dans l'Exemple 1 en remplaçant au stade A le 1-bromo-2-méthylbenzène par le 1-bromo-2-chlorobenzène et en remplaçant au stade F le composé obtenu dans la Préparation 1 par le composé obtenu dans la Préparation 14.

### EXEMPLE 56 : N-{3-[(Phénylthio)méthyl)-2-cycloheptén-1-yl}-N,N-bis(3-pyridinyl-méthyl)amine

On procède comme au stade F de l'Exemple 1 à partir du composé obtenu dans l'Exemple 42 en remplaçant le composé obtenu dans la Préparation 1 par le nicotinaldehyde.

### EXEMPLE 57 : 4-{[5-(2-{[3-(3-Chlorophényl)-2-cycloheptèn-1-yl]amino}éthyl)-1H-imidazol-1-yl]méthyl}benzonitrile

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 12.

### EXEMPLE 58 : 4-[{[3-(3-Chlorophényl)-2-cycloheptèn-1-yl]amino}(1H-imidazol-5-yl)méthyl]benzonitrile

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 15.

### EXEMPLE 59 : 4-[{[3-(3-Chlorophényl)-2-cycloheptèn-1-yl]amino}(1-méthyl-1H-imidazol-5-yl)méthyl]benzonitrile

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 16.

### EXEMPLE 60 : 4-[2-{[3-(3-Chlorophényl)-2-cycloheptèn-1-yl]amino}-2-(1H-imidazol-5-yl)éthyl]benzonitrile

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 17.

### EXEMPLE 61: 4-[2-{[3-(3-Chlorophényl)-2-cycloheptèn-1-yl]amino}-2-(1-méthyl-1H-imidazol-5-yl)éthyl]benzonitrile

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 18.

### EXEMPLE 62 : 3-(3-Chlorophényl)-N-[(4-chlorophényl)(1H-imidazol-5-yl)méthyl]-2-cycloheptèn-1-amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 19.

### EXEMPLE 63 : 3-(3-Chlorophényl)-N-[(4-chlorophényl)(1-méthy]-1H-imidazol-5-yl)méthyl]-2-cycloheptèn-1-amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 20.

### EXEMPLE 64 : 3-(3-Chlorophényl)-N-[2-(4-chlorophényl)-1-(1H-imidazol-5-yl)éthyl]-2-cycloheptèn-1-amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 21.

### EXEMPLE 65 : 3-(3-Chlorophényl)-N-[2-(4-chlorophényl)-1-(1-méthyl-1H-imidazol-5-yl)éthyl]-2-cycloheptèn-1-amine

On procède comme dans l'Exemple 1 en remplaçant au stade A le 1-bromo-2-méthylbenzène par le 1-bromo-3-chlorobenzène et en remplaçant au stade F le composé obtenu dans la Préparation 1 par le composé obtenu dans la Préparation 22.

### ETUDE PHARMACOLOGIQUE

### EXEMPLE A : Tests enzymatiques

Les deux enzymes FTase et GGTase-I ont été purifiées à partir de cerveaux de rat. Après broyage et centrifugation, le surnageant est précipité au sulfate d'ammonium à 30% et le surnageant correspondant soumis à une autre précipitation au sulfate d'ammonium à 50%. Le culot est alors passé sur une colonne de phényl agarose et les fractions collectées après élution au chlorure de sodium sont évaluées pour leur teneur enzymatique selon la méthode de "scintillation proximity assay" décrite ci-dessous. Les fractions correspondant à l'une ou l'autre des deux enzymes sont alors regroupées et congelées à -80°C jusqu'à utilisation.

Le dosage de l'activité enzymatique de la FTase est réalisé en plaques de 96 puits par la méthode radioactive de scintillation proximity assay. Le substrat accepteur composé de la séquence carboxyterminale de la lamine B (YRASNRSCAIM) couplée à la biotine est incubé en présence du substrat donneur radiomarqué (le [³H]farnésyl pyrophosphate), et de diverses concentrations de composés à tester dans le DMSO. La réaction est initiée à 37°C en ajoutant l'enzyme FTase pour une durée d'une heure, puis stoppée avec un tampon approprié contenant une suspension de billes imprégnées de scintillant. Ces billes sont de plus couplées à la streptavidine pour piéger, par couplage à la biotine, le peptide susceptible d'être farnésylé et mettre ainsi en contact le farnésyl radiomarqué avec le scintillant. Les plaques sont lues dans un compteur pour radioactivité et les données converties en pourcentages du contrôle pour exprimer les résultats sous forme de concentration du produit testé entraînant 50% d'inhibition de la farnésylation (IC50).
Pour la GGTase-I, un test équivalent a été utilisé en remplaçant le substrat accepteur par la séquence biotinylée TKCVIL et le substrat donneur par du [³H]géranylgéranyl pyrophosphate.

### Résultats :

Les composés de la présente invention possèdent des IC50 de l'ordre du nanomolaire vis à vis de la FTase, révélant leur caractère de puissant inhibiteur de cet enzyme, et présentent une sélectivité importante par rapport à la GGTase-I, les IC50 étant alors seulement de l'ordre du micromolaire.

### EXEMPLE B : Tests de prolifération cellulaire :

a) La lignée RAT2 de fibroblastes de rat et un transfectant correspondant à l'insertion du gène v-H-ras ont été utilisés pour tester la puissance cellulaire des produits revendiqués. Les cellules RAT2 permettent de caractériser la toxicité intrinsèque du produit testé, alors que les cellules transfectées qui exhibent une morphologie altérée et une vitesse de croissance plus rapide, servent à mesurer l'effet spécifique recherché sur la FTase intracellulaire.

Les cellules parentales et transfectées sont ensemencées en plaques 96 puits pour la culture cellulaire en présence de milieu contenant 10% de sérum. Vingt-quatre heures après, les produits à tester sont ajoutés dans le même milieu sur une période de quatre jours et la quantité finale de cellules est estimée indirectement par la méthode de viabilité cellulaire au bromure de 3-(4,5-diméthylthiazol-2-yl)-2,5-diphényltétrazolium (MTT).

### Résultats :

Un ralentissement de la croissance des cellules transfectées par v-H-ras est observé pour les composés de l'invention dans la gamme du nanomolaire. Cet effet traduisant le retour des cellules transfectées aux caractéristiques de croissance de la lignée parentale, s'accompagne aussi d'une réversion de la morphologie des transfectants vers le phénotype parental (étalement et perte de réfringence). Plusieurs unités logarithmiques séparent cet effet spécifique de l'effet cytotoxique observé sur les cellules RAT2 dans la gamme des micromolaires, le différentiel le plus favorable étant d'au moins quatre unités pour les produits les plus actifs.

b) Des tests complémentaires sur des lignées de carcinomes humains issues de biopsies cliniques sont effectués. Les lignées utilisées proviennent toutes de l'ATCC (American Type Culture Collection) et le test est réalisé en plaques 96 puits sur une durée de contact avec le produit correspondant à quatre temps de doublement.

### Résultats :

Une observation au microscope et un dénombrement indirect par la méthode du MTT ont permis de mettre en évidence une activité anti-proliférative avec des IC50s de l'ordre d'une centaine de nanomolaires pour les composés de l'invention sur la lignée EJ138, un carcinome de vessie exhibant une mutation de la protéine H-Ras. Cette inhibition s'accompagne d'un effet sur la morphologie des cellules similaire à celui observé sur les transfectants v-H-ras de rat.

### EXEMPLE C: Test de prénylation in vitra de la protéine Ras :

Les cellules fibroblastiques de rat transfectées par v-H-ras et les cellules de carcinome vésical EJ138 exhibant une mutation H-Ras sont ensemencées à forte densité, puis traitées vingt-quatre heures après et pendant quarante-huit heures avec différentes concentrations de composés à tester. Les lysats cellulaires sont déposés sur un gel pour électrophorèse et les protéines séparées sont transférées pour une exploitation en Western blot avec un anticorps dirigé contre la protéine Ras reconnaissant les formes prénylées ou non.

### Résultats :

On observe avec les composés de l'invention une modification de la farnésylation de Ras avec un effet moitié de l'ordre de 10 nM, coïncidant avec la puissance sur l'enzyme FTase purifiée.

### EXEMPLE D : Test de croissance en agar :

Les cellules sont ensemencées en présence de sérum et de diverses concentrations de composés à tester dans de l'agar pour évaluer leur croissance indépendamment du substrat. Dans ces conditions de croissance dite clonogénique, les cellules RAT2 restent à l'état de cellules isolées et viables pendant la durée de l'expérience (deux semaines). Par contre, les cellules transfectées par v-H-ras forment des colonies multicellulaires que l'on peut dénombrer et dont on peut mesurer la taille par analyse d'images.

### Résultats :

Les composés de l'invention inhibent la formation d'agrégats avec une IC50 de l'ordre de 10 nM, sans exercer un effet cytotoxique puisque la majorité des cellules transfectées traitées par des concentrations supérieures à l'IC50 restent sous forme de cellules isolées et viables comme les cellules RAT2 parentales non traitées.

### EXEMPLE E : Test d'angiogenèse in vitro :

Ce test consiste à cultiver dans un milieu parfaitement défini et sans sérum des fragments d'aorte de rat dans un gel tridimensionnel de collagène selon une méthode décrite dans Lab Invest 1990, 63, 115-122. Une arborisation vasculaire se met en place dès le troisième jour de culture, précédée par une émigration importante de fibroblastes individualisés.

### Résultats :

Dans ces conditions de culture et après cinq jours de contact avec les composés de l'invention, un effet sélectif sur l'inhibition de l'excroissance cellulaire est observé : seules les cellules endothéliales sont affectées avec une IC50 de l'ordre d'une centaine de nanomolaires.

### EXEMPLE F : Composition Pharmaceutique

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I): 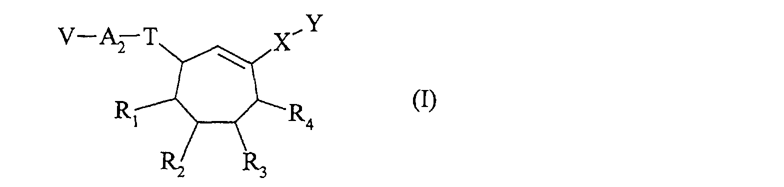 dans laquelle :
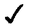 X représente une liaison ou un groupement choisi parmi alkylène, CO, S(O)ₙ, ∗-S(O)ₙ-A₁-, ∗-CO-A₁-, -A₁-S(O)ₙ-A'₁-, et -A₁-CO-A'₁- (dans lesquels A₁ et A'₁, identiques ou différents, représentent un groupement alkylène et n vaut 0, 1 ou 2), le symbole "∗" représentant le point de rattachement de ces groupements au cycloheptène,
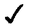 Y représente un groupement aryle, hétéroaryle, cycloalkyle ou hétérocycloalkyle, chacun de ces groupements étant non substitué ou substitué par un ou plusieurs, identiques ou différents, groupements R₈,
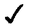 R₁, R₂, R₃ et R₄ représentent indépendamment un atome d'hydrogène ou un groupement aryle, hétéroaryle, cycloalkyle ou hétérocycloalkyle, chacun de ces groupements étant non substitué ou substitué par un groupement R₈,
ou bien R₁, R₂, R₃ et R₄, pris deux à deux, forment ensemble une liaison,
ou bien, R₁ et R₂, ou R₂ et R₃ ou R₃ et R₄ pris deux à deux avec les atomes de carbone auxquels ils sont liés, forment un cycle benzénique fusionné ou un hétérocycle fusionné aromatique ou partiellement hydrogéné, de 5 ou 6 chaînons contenant 1 ou 2 hétéroatomes choisis parmi azote, oxygène et soufre, sachant qu'un seul cycle peut être fusionné sur la structure à 7 chaînons,
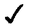 T représente un groupement -CH(R₅)-, -N(R₅)- ou ∗-N(R₅)CO- (où R₅ représente un atome d'hydrogène ou un groupement alkyle, aryle, hétéroaryle, arylalkyle ou hétéroarylalkyle, chacun de ces groupements étant non substitué ou substitué par un ou plusieurs, identiques ou différents, groupements R₇), le symbole "∗" représentant le point de rattachement de ce groupement au cycloheptène,
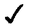 V représente un atome d'hydrogène ou un groupement aryle ou hétéroaryle, chacun de ces groupements étant non substitué ou substitué par un ou plusieurs, identiques ou différents, groupements R₇,
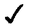 A₂ représente un groupement [C(R₆)(R'₆)]ₚ où p vaut 0, 1, 2, 3 ou 4 lorsque T représente un groupement -CH(R₅)- ou *-N(R₅)CO- ou p vaut 1, 2, 3 ou 4 lorsque T représente un groupement -N(R₅)-, et R₆ et R'₆, identiques ou différents, représentent un atome d'hydrogène ou un groupement alkyle, alkényle, alkynyle, aryle éventuellement substitué, hétéroaryle éventuellement substitué, hétérocycloalkyle éventuellement substitué, arylalkyle éventuellement substitué, hétéroarylalkyle éventuellement substitué, hétérocycloalkylalkyle éventuellement substitué, R₉ ou alkyle substitué par un groupement R₉ (où R₉ représente un groupement -OR₅, -N(R₅)(R'₅), -S(O)ₘR₅, -CON(R₅)(R'₅), -N(R₅)COR'₅, -N(R₅)SO₂R'₅, -SO₂N(R₅)(R'₅), -N(R₅)COO(R'₅), m étant égal à 0, 1 ou 2, et R'₅ peut prendre toutes les valeurs de R₅),
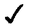 R₇ représente un atome d'halogène ou un groupement alkyle, alkoxy, hydroxy, mercapto, alkylthio, cyano, amino (éventuellement substitué par un ou deux groupements alkyle), nitro, carboxy, alkoxycarbonyle, aminocarbonyle (éventuellement substitué par un ou deux groupements alkyle), carbamoyle, aryle non substitué ou substitué, arylalkyle non substitué ou substitué, hétéroaryle non substitué ou substitué, hétéroarylalkyle non substitué ou substitué, cycloalkyle non substitué ou substitué, cycloalkylalkyle non substitué ou substitué, hétérocycloalkyle non substitué ou substitué ou hétérocycloalkylalkyle non substitué ou substitué,
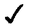 R₈ représente un atome d'halogène, ou un groupement oxo, hydroxy, cyano, nitro, carboxy, alkoxycarbonyl, perhalogénoalkyle ou un groupement -U-R₈₀, ou -A₈₀-U-R₈₀ (dans lesquels A₈₀ représente un groupement alkylène ; U représente une liaison, un atome d'oxygène, ou un groupement choisi parmi NH, S(O)ₘ, NHCO, CONH, SO₂NH, et NHSO₂, m étant égal à 0, 1, ou 2 ; et R₈₀ est un groupement choisi parmi alkyle, aryle, arylalkyle, hétéroaryle et hétéroarylalkyle),
étant entendu que :
- le terme alkyle désigne un groupement linéaire ou ramifié, contenant de 1 à 6 atomes de carbone,
- le terme alkylène désigne un groupement bivalent linéaire ou ramifié contenant de 1 à 6 atomes de carbone,
- le terme cycloalkyle désigne un groupement cyclique saturé contenant de 3 à 8 atomes de carbone,
- le terme hétérocycloalkyle désigne un groupement cyclique saturé ou partiellement insaturé de 5 à 7 chaînons contenant de 1 à 3 hétéroatomes choisis parmi azote, oxygène et soufre,
- le terme aryle désigne un groupement phényle ou naphtyle,
- le terme hétéroaryle désigne un groupement mono ou bicyclique, aromatique ou contenant au moins un cycle aromatique, de 5 à 11 chaînons, contenant de 1 à 5 hétéroatomes choisis parmi azote, oxygène et soufre,
- le terme substitué affecté aux expressions aryle, hétéroaryle, cycloalkyle, et hétérocycloalkyle signifie que ces groupements peuvent être substitués par un ou plusieurs groupements, identiques ou différents, choisis parmi cyano, alkylcarbonyle, aminocarbonyle (éventuellement substitué par un ou deux groupements alkyle), ou atomes d'halogène,
- le terme substitué affecté aux expressions arylalkyle, hétéroarylalkyle, cycloalkylalkyle, et hétérocycloalkylalkyle signifie que la partie cyclique de ces groupements peut être substituée par un ou plusieurs groupements, identiques ou différents, choisis parmi oxo, cyano, alkylcarbonyle, aminocarbonyle (éventuellement substitué par un ou deux groupements alkyle), ou atomes d'halogène,
leurs énantiomères, diastéréoisomères, ainsi que leurs sels d'addition à un acide ou à une base pharmaceutiquement acceptable.

2. Composés de formule (I), selon la revendication 1 pour lesquels X représente une liaison, leurs énantiomères, diastéréoisomères ainsi que leurs sels d'addition à un acide ou à une base pharmaceutiquement acceptable.

3. Composés de formule (I), selon la revendication 1 pour lesquels Y représente un groupement aryle éventuellement substitué par R₈, leurs énantiomères, diastéréoisomères ainsi que leurs sels d'addition à un acide ou à une base pharmaceutiquement acceptable.

4. Composés de formule (I), selon la revendication 1 pour lesquels R₁, R₂, R₃ et R₄ représentent chacun un atome d'hydrogène, leurs énantiomères, diastéréoisomères ainsi que leurs sels d'addition à un acide ou à une base pharmaceutiquement acceptable.

5. Composés de formule (I), selon la revendication 1 pour lesquels T représente un groupement -N(R₅)-, leurs énantiomères, diastéréoisomères ainsi que leurs sels d'addition à un acide ou à une base pharmaceutiquement acceptable.

6. Composé de formule (I) selon la revendication 5 pour lesquels T représente un groupement -NH-, leurs énantiomères, diastéréoisomères ainsi que leurs sels d'addition à un acide ou à une base pharmaceutiquement acceptable.

7. Composés de formule (I), selon la revendication 1 pour lesquels A₂ représente un groupement -CH₂- ou -CH₂-CH₂-, leurs énantiomères, diastéréoisomères ainsi que leurs sels d'addition à un acide ou à une base pharmaceutiquement acceptable.

8. Composés de formule (I), selon la revendication 1 pour lesquels A₂ représente un groupement (4-cyanophényl)méthylène, (4-chlorophényl)méthylène, (4-cyanobenzyl) méthylène ou (4-chlorobenzyl)méthylène, leurs énantiomères, diastéréoisomères ainsi que leurs sels d'addition à un acide ou à une base pharmaceutiquement acceptable.

9. Composés de formule (I), selon la revendication 1 pour lesquels V représente un groupement hétéroaryle éventuellement substitué par R₇, leurs énantiomères, diastéréoisomères ainsi que leurs sels d'addition à un acide ou à une base pharmaceutiquement acceptable.

10. Composés de formule (I), selon la revendication 1 pour lesquels V représente un groupement imidazolyle éventuellement substitué par R₇, leurs énantiomères, diastéréoisomères ainsi que leurs sels d'addition à un acide ou à une base pharmaceutiquement acceptable.

11. Composés de formule (I), selon l'une quelconque des revendications 9 et 10 pour lesquels R₇ représente un groupement arylalkyle non substitué ou substitué, ou hétéroaryle non substitué ou substitué, leurs énantiomères, diastéréoisomères ainsi que leurs sels d'addition à un acide ou à une base pharmaceutiquement acceptable.

12. Composés de formule (I), selon la revendication 1 pour lesquels X représente une liaison ; Y représente un groupement aryle éventuellement substitué par R₈; R₁, R₂, R₃ et R₄ représentent chacun un atome d'hydrogène ; T représente un groupement -NH- ; A₂ représente un groupement -CH₂, -CH₂-CH₂-, (4-cyanophényl)méthylène, (4-chlorophényl)méthylène, (4-cyanobenzyl)méthylène ou (4-chlorobenzyl)méthylène ; et V représente un groupement hétéroaryle éventuellement substitué par R₇, leurs énantiomères, diastéréoisomères ainsi que leurs sels d'addition à un acide ou à une base pharmaceutiquement acceptable.

13. Composés de formule (I), selon la revendication 1 pour lesquels X représente une liaison ; Y représente un groupement aryle éventuellement substitué par R₈ ; R₁, R₂, R₃ et R₄ représentent chacun un atome d'hydrogène ; T représente un groupement -NH- ; A₂ représente un groupement -CH₂, -CH₂-CH₂-, (4-cyanophényl)méthylène, (4-chlorophényl)méthylène, (4-cyanobenzyl)méthylène ou (4-chlorobenzyl)méthylène; et V représente un groupement imidazolyle éventuellement substitué par R₇, leurs énantiomères, diastéréoisomères ainsi que leurs sels d'addition à un acide ou à une base pharmaceutiquement acceptable.

14. Composés de formule (I), selon la revendication 1 qui sont le (R,S)-4-{[5-({[3-(2-méthylphényl)-2-cycloheptèn-1-yl]amino}méthyl)-1*H*-imidazol-1-yl]méthyl}benzonitrile, le 4-{[5-({[3-(3-méthylphényl)-2-cycloheptèn-1-yl]amino}méthyl)-1*H*-imidazol-1-yl] méthyl}benzonitrile et le 4-{[5-({[3-(3-chlorophényl)-2-cycloheptèn-1-yl]amino}méthyl)-1*H*-imidazol-1-yl]méthyl}benzonitrile.

15. Procédé de préparation des composés de formule (I), selon la revendication 1, **caractérisé en ce que** l'on utilise comme produit de départ un composé de formule (II) : 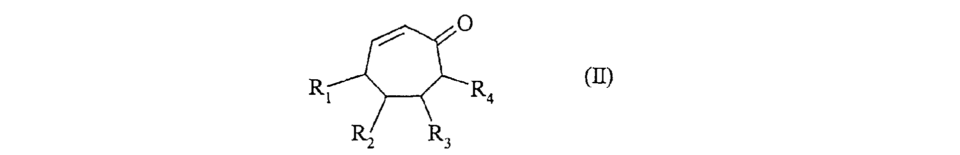 dans laquelle R₁, R₂, R₃ et R₄ sont tels que définis dans la formule (I),
dont la fonction carbonyle réagit avec un composé organométallique de formule Li-X-Y dans laquelle X et Y sont tels que définis dans la formule (I), pour conduire à un composé de formule (III) : 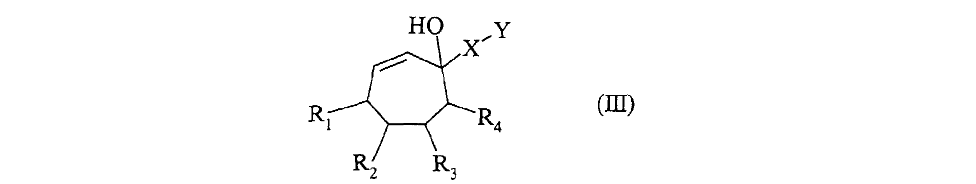 dans laquelle R₁, R₂, R₃, R₄, X et Y sont tels que définis précédemment,
qui subit une réaction d'isomérisation en milieu acide, pour conduire à un composé de formule (IV) : 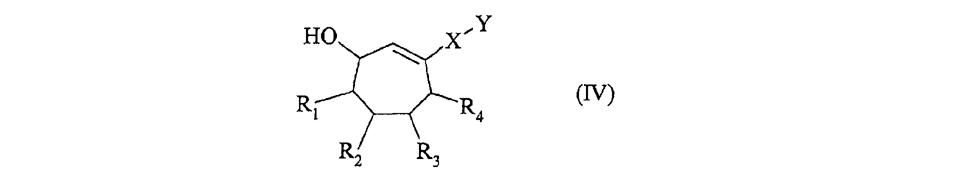 dans laquelle R₁, R₂, R₃, R₄, X et Y sont tels que définis précédemment,
composé de formule (IV), qui après transformation de la fonction hydroxyle en groupe partant, est soumis :
- soit à l'introduction d'une fonction réactive par action d'un dérivé silylé tel que Me₃SiCN, CH₂=CH-CH₂-SiMe₃ ou CH₂=C(Oalk)(OSiMe₃) où alk représente un groupement alkyle, puis à la condensation des groupements appropriés pour conduire à un composé de formule (I/a): 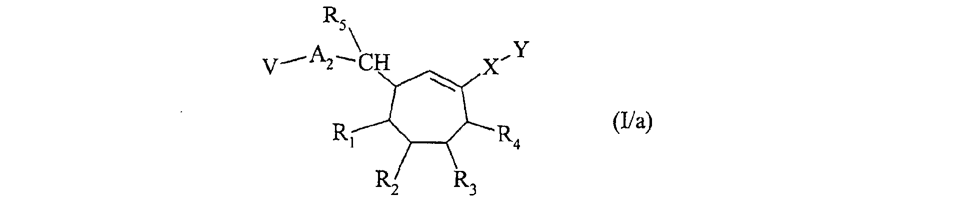 cas particulier des composés de formule (I) pour lequel R₁, R₂, R₃, R₄, R₅, A₂, V, X et Y sont tels que définis précédemment,
- soit à l'action de l'azidure de sodium, pour conduire, après hydrolyse en présence de triphénylphosphine, à une amine de formule (V) : 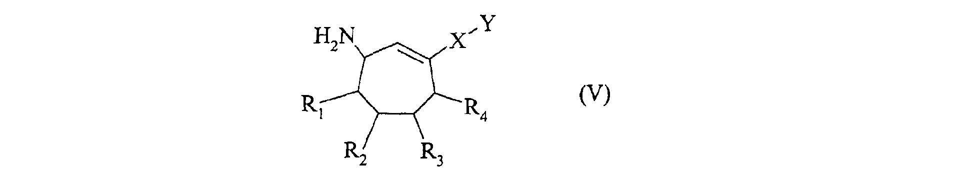
dans laquelle R₁, R₂, R₃, R₄, X et Y sont tels que définis précédemment,
composé de formule (V) qui est condensé sur un aldéhyde de formule : 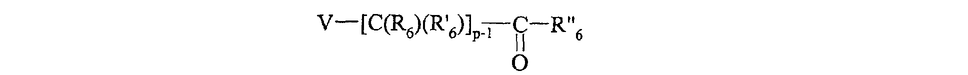 dans laquelle V est tel que défini précédemment, R₆ et R'₆ sont tels que définis dans la formule (I), R"₆ représente les mêmes atomes ou groupements que ceux définis par R₆ ou R'₆, et p est égal à 1, 2, 3 ou 4, pour conduire à un composé de formule (I/b) : 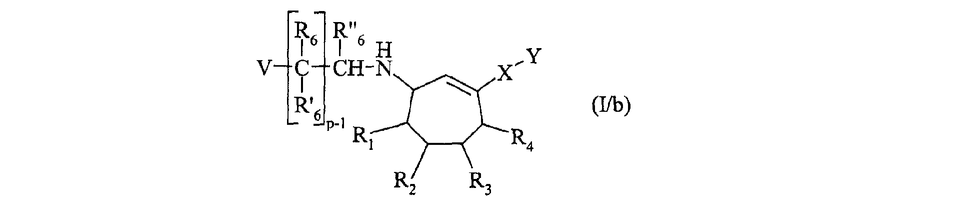 cas particulier des composés de formule (I) pour lequel R₁, R₂, R₃, R₄, R₆, R'₆, R"₆, V, X, Y et p sont tels que définis précédemment,
[certains composés de formule (I/b) pouvant par ailleurs être obtenus à partir du composé de formule (II') : 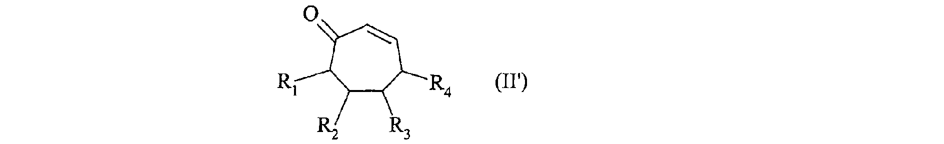 dans laquelle R₁, R₂, R₃ et R₄ sont tels que définis précédemment,
que l'on soumet, après formation de l'éther silylé correspondant, à l'action, en milieu basique fort, d'un composé Y-X'-CHO dans lequel Y est tel que défini précédemment et X' représente une liaison ou un groupement alkylène, pour conduire, après déprotection au composé de formule (III') ; 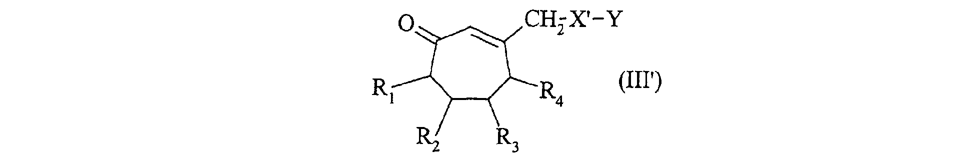 dans laquelle R₁, R₂, R₃, R₄, X' et Y sont tels que définis précédemment,
sur lequel on condense une amine de formule V-A₂-NH₂ dans laquelle V et A₂ sont tels que définis précédemment, pour obtenir le composé de formule (I/bₐ), cas particulier des composés de formule (I/b): 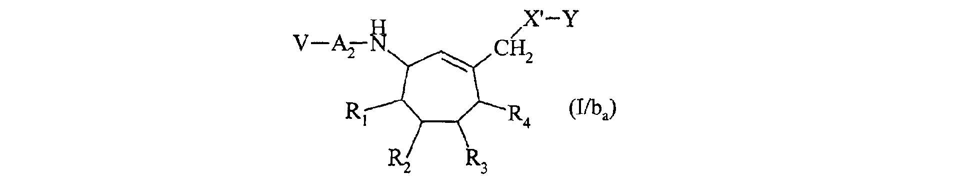 dans laquelle R₁, R₂, R₃, R₄, V, A₂, X' et Y sont tels que définis précédemment],
ou composé de formule (V) qui est soumis à une réaction d'acylation à l'aide d'un composé de formule V-A₂-CO-Hal dans laquelle V et A₂ sont tels que définis dans la formule (I) et Hal représente un atome d'halogène, ou à un couplage avec un acide carboxylique de formule V-A₂-COOH dans laquelle V et A₂ sont tels que définis dans la formule (I), pour conduire à un composé de formule (I'/b) : 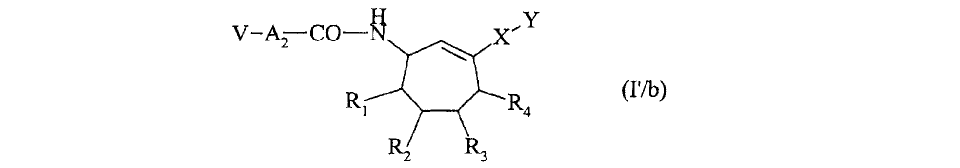 dans laquelle R₁, R₂, R₃, R₄, A₂, V, X et Y sont tels que définis précédemment,
composés (I/b) et (I'/b) qui peuvent être soumis au même type de condensation que précédemment, sur un aldéhyde de formule R"₅CHO, ou sur un halogénure d'acyle de formule R"₅-CO-Hal, ou encore sur un acide carboxylique de formule R"₅-COOH, dans lesquelles R"₅ peut prendre toutes les valeurs de R₅ à l'exception de l'atome d'hydrogène pour conduire à un composé de formule (I/c): 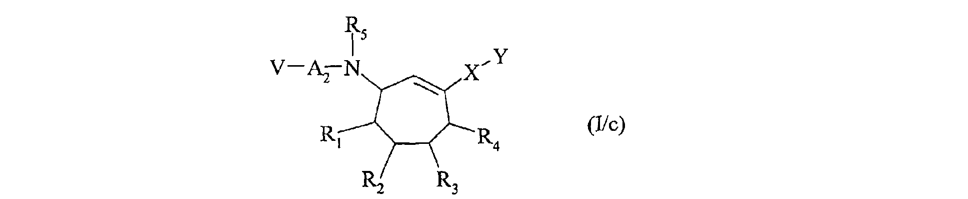 cas particulier des composés de formule (I) pour lequel R₁, R₂, R₃, R₄, R₅, A₂, V, X et Y sont tels que définis précédemment,
composés de formule (I/a), (I/b), (I'/b) et (I/c) formant la totalité des composés de formule (I),
- qui peuvent être, le cas échéant, purifiés selon une technique classique de purification,
- dont on sépare, le cas échéant, les isomères selon une technique classique de séparation,
- que l'on transforme, si on le souhaite, en ses leurs d'addition à un acide ou à une base pharmaceutiquement acceptable.

16. Compositions pharmaceutiques contenant comme principe actif au moins un composé selon l'une quelconque des revendications 1 à 14, seul ou en combinaison avec un ou plusieurs excipients ou véhicules inertes, non toxiques, pharmaceutiquement acceptables.

17. Compositions pharmaceutiques selon la revendication 16 contenant au moins un principe actif selon l'une quelconque des revendications 1 à 14 utiles pour la fabrication de médicaments utiles dans le traitement des maladies cancéreuses.

18. Compositions pharmaceutiques selon la revendication 16 contenant au moins un principe actif selon l'une quelconque des revendications 1 à 14 utiles pour la fabrication de médicaments utiles dans le traitement de la resténose après angioplastie ou chirurgie vasculaire, et de la neurofibromatose de type I.
